(19) Europäisches Patentamt
European Patent Office
Office européen des brevets

(11) EP 1 652 917 A1

(12) EUROPEAN PATENT APPLICATION
published in accordance with Art. 158(3) EPC

(43) Date of publication:
**03.05.2006 Bulletin 2006/18**

(21) Application number: **04771250.0**

(22) Date of filing: **29.07.2004**

(51) Int Cl.:
***C12N 15/09*** (1990.01) ***C12Q 1/02*** (1980.01)
***A61K 48/00*** (1990.01) ***A61P 9/10*** (2000.01)
***A61P 35/00*** (2000.01)

(86) International application number:
**PCT/JP2004/011223**

(87) International publication number:
**WO 2005/010185 (03.02.2005 Gazette 2005/05)**

(84) Designated Contracting States:
**AT BE BG CH CY CZ DE DK EE ES FI FR GB GR HU IE IT LI LU MC NL PL PT RO SE SI SK TR**

(30) Priority: **29.07.2003 JP 2003202863**
**16.03.2004 JP 2004075115**

(71) Applicants:
- **KYOWA HAKKO KOGYO CO., LTD.**
  **Chiyoda-ku,**
  **Tokyo 100-8185 (JP)**
- **Nagai, Ryozo**
  **X (JP)**
- **Manabe, Ichiro**
  **Taito**
  **Tokyo 110-0008 (JP)**

(72) Inventors:
- **NAGAI, Ryozo**
  **Tokyo 113-0033 (JP)**
- **MANABE, Ichiro**
  **Tokyo 110-0008 (JP)**
- **ISHIHARA, Atsushi**
  **c/p Pharmaceutical res center**
  **Shizuoka 411-8731 (JP)**
- **TOTTORI, Tsuneaki**
  **Osaka 565-2851 (JP)**

(74) Representative: **Sternagel, Fleischer, Godemeyer & Partner**
**Patentanwälte,**
**An den Gärten 7**
**51491 Overath (DE)**

# (54) RNA CAPABLE OF INHIBITING EXPRESSION OF KLF5 GENE

(57) An RNA capable of suppressing the expression of KLF5 gene, which comprises a sequence consisting of 15 to 30 contiguous nucleotides of KLF5 mRNA and a sequence complementary to the sequence, and which has been designed from the nucleotide sequence of Kruppel-like factor 5 (KLF5) cDNA. Specifically, a double-stranded RNA having a strand of a sequence shown in any one of SEQ ID NOS: 2 to 16 and a strand of a sequence complementary to the sequence, in which 2 uridylic acids are added to the 3'-terminus of each of the strands. By transfecting the RNA or a vector for expression of the RNA into cells, the expression of KLF5 gene in the cells can be suppressed. The RNA or a vector for expression of the RNA can be used as a therapeutic agent for cardiovascular disease or cancer.

EP 1 652 917 A1

## Description

TECHNICAL FIELD

The present invention relates to RNA that is capable of suppressing the expression of KLF5 gene.

BACKGROUND ART

The Kruppel-like factor (hereinafter abbreviated as KLF) family is a family of transcriptional factors, which is characterized in that it has zinc finger motifs at the C-terminus thereof, and examples thereof that have been known include KLF1, KLF2, KLF3, KLF4, KLF5, KLF6, KLF7, KLF8, KLF9, KLF10, KLF11, KLF12, KLF13, KLF14, KLF15, and KLF 16. It has been reported that, in mammals, the KLF family plays an important role in differentiation of various types of tissues or cells, such as erythrocytes, vascular endothelial cells, smooth muscle, skin, or lymphocytes, and also in formation of the pathologic conditions of various types of diseases such as cancer, cardiovascular disease, hepatocirrhosis, renal disease, or immune-mediated disease (J. Biol. Chem., 276, 34355-34358, 2001; Genome Biol., 4, 206, 2003).

Among the KLF family members, KLF5 is also referred to as BTEB2 (basic transcriptional element binding protein 2) or IKLF (intestinal-enriched Kruppel-like factor). The expression of KLF5 in vascular smooth muscle is controlled at the development stage thereof. KLF5 is highly expressed in the vascular smooth muscle of a fetus, whereas its expression is not found in the vascular smooth muscle of a healthy adult. In addition, in the case of the smooth muscle of intima of a blood vessel regenerated after denudation by a balloon catheter, KLF5 is highly expressed. Also, in the smooth muscle of lesions due to arteriosclerosis or restenosis, KLF5 is expressed (Circulation, 102, 2528-2534, 2000).

The vascular smooth muscle cells of lesions such as an arteriosclerosis lesion or a restenosis site formed after percutaneous transluminal coronary angioplasty are activated. Such vascular smooth muscle cells exhibit the disappearance of myofilaments, stimulation of protein synthesis, growth ability, and migration ability. Thus, such vascular smooth muscle cells have been transformed, so as to have the same characteristics as those of the vascular smooth muscle of an embryo (embryonic type). In smooth muscle cells, 3 types of isoforms of a myosin heavy chain, such as SM1, SM2, and SMemb, are present. However, as the vascular smooth muscle is transformed to a fetal type, SM2 disappears, and induction of the expression of SMemb is observed. KLF5 binds to the transcriptional regulatory sequence of the SMemb gene, so as to activate the transcription thereof (refer to Non-Patent Document 4). Further, it has been reported that KLF5 activates the transcription of genes associated with the characteristics of a blood vessel or vascularization, such as platelet-derived growth factor A chain (hereinafter referred to as PDGF-A), transforming growth factor (TGF)-β, vascular endothelial growth factor (VEGF) receptor, inducible nitric oxide synthase (iNOS), plasminogen activator inhibitor (PAI) -1, or transcription factor Egr (early growth response) -1 (Nat. Med., 8, 856-863, 2002; Ann. N. Y. Acad. Sci., 947, 56-66, 2001).

Moreover, it has been reported that, in KLF5 gene hetero-knockout mice, the growth of vascular smooth muscle, endothelial proliferation, vascularization, formation of granulation in the adventitia of a blood vessel, cardiac hypertrophy, and the development of fibrotic cardiomyopathy, which are caused by physical loading on a cardiovascular system or angiotensin II, are significantly suppressed (Nat. Med., 8, 856-863, 2002).

Thus, the KLF5 gene is not only associated with transformation of smooth muscle, but it is also a transcriptional factor associated with formation of a broad range of pathologic conditions of the cardiovascular system. The expression level of the gene is extremely important for the expression of the functions thereof. KLF5 is associated with formation of the pathologic conditions of cardiovascular diseases such as arteriosclerosis or cardiac hypertrophy, or angiogenesis-related diseases such as cancer. Thus, it is anticipated that a pharmaceutical agent useful for the treatment or prevention of the aforementioned diseases be developed by suppressing the expression of the KLF gene. However, to date, a pharmaceutical agent for effectively suppressing the expression of the KLF family genes has not yet been known.

On the other hand, it has been reported that RNA interference (hereinafter referred to as RNAi) is a phenomenon whereby when double-stranded RNA having a sequence identical to that of a target gene is introduced into nematode, the expression of the target gene is specifically suppressed (Nature, 391, 806-811, 1998).

It is considered that the introduced double-stranded RNA is decomposed to double-stranded RNA having a length of 21 to 23 nucleotides, that a protein complex then binds to this short double-stranded RNA, that it recognizes mRNA having the same sequence and then cleaved it, and thus that RNAi takes place. Tuschl et al. have found that even when a double-stranded RNA having a length of 21 to 23 nucleotides is introduced into drosophila, instead of a long double-stranded RNA, the expression of a target gene is suppressed. This was named short interfering RNA (siRNA) (WO01/75164). When there is a mismatch between the sequence of siRNA and that of a target gene, the effect of suppressing expression is significantly reduced. The length consisting of 21 nucleotides brings on the highest effect. When such double-stranded RNA has a structure with protrusive termini obtained by adding nucleotides to 3'-termini of both strands, it provides a higher effect than that of double-stranded RNA having blunt ends (WO02/44321).

In the case of mammalian cells, when a long double-stranded RNA has been introduced, suppression of the expression of all genes and apoptosis have taken place as a result of the functions of virus defense mechanism, and thus suppression of a specific gene was impossible. However, it has been found that when siRNA having a length of 20 to 29 nucleotides is used, such a reaction does not take place, and that the expression of a specific gene can be suppressed. Among others, siRNA having 21 to 25 nucleotides has a high effect of suppressing expression (Nature, 411, 494-498, 2001; Nat. Rev. Genet., 3, 737-747, 2002; Mol. Cell, 10, 549-561, 2002; Nat. Biotechnol., 20, 497-500, 2002).

It has been reported that in RNAi, the effect of double-stranded RNA to suppress the expression of a target gene is significantly higher than that of single-stranded antisense RNA (Nature, 391, 806-811, 1998; Mol. Cell, 10, 549-561, 2002). In addition, it has also been reported that not only double-stranded RNA, but also single-stranded RNA forming a hairpin structure as a result of intramolecular hybridization, exhibits RNAi, as with siRNA (Proc. Natl. Acad. Sci. USA, 99, 6047-6052, 2002).

RNAi has been verified not only *in vitro* tests but also in *in vivo* tests. The effect of RNAi using siRNA with a length of 50 bp or less on fetal animals (WO02/132788) and the effect thereof on adult mice (WO03/10180) have been reported. Moreover, when siRNA is intravenously administered to a fetal mouse, the effect of suppressing expression was found in various organs such as kidney, spleen, lung, pancreas, and liver (Nat. Genet. 32, 107-108, 2002). Furthermore, it has been reported that when siRNA is directly administered to brain cells, it acts on them (Nat. Biotechnol., 20, 1006-1010, 2002). However, RNAi using siRNA on KLF5 or other KLF family genes has not yet been reported to date.

DISCLOSURE OF THE INVENTION

It is an object of the present invention to find RNA that is capable of suppressing the expression of KLF5 gene. Such RNA suppresses the expression of the KLF gene, so as to inhibit the functions of KLF5 as a transcriptional factor. Thus, such RNA can be used as a therapeutic or preventive agent causing few side effects, which is used for diseases such as cardiovascular disease or cancer, of which diseases KLF5 is associated with formation of the pathologic conditions.

The present inventors have conducted intensive studies, and as a result, they have completed the following invention. That is to say, the present invention relates to the following (1) to (13):

(1) An RNA capable of suppressing the expression of KLF5 gene, which comprises a sequence consisting of 15 to 30 contiguous nucleotides of KLF5 mRNA and a sequence complementary to the sequence.
(2) The RNA according to (1), wherein the KLF5 mRNA is human KLF5 mRNA or mouse KLF5 mRNA.
(3) The RNA according to (1) or (2), wherein the RNA is a double-stranded RNA consisting of a strand of sequence consisting of 15 to 30 contiguous nucleotides of KLF5 mRNA and a strand of sequence complementary to the sequence, in which 1 to 6 nucleotides are added to the 3'-terminus of each of the strands.
(4) The RNA according to (1) or (2), wherein the RNA is an RNA forming a hairpin structure, which is obtained by ligating an RNA having a sequence consisting of 15 to 30 contiguous nucleotides of the KLF5 mRNA to an RNA having a sequence complementary to the sequence via a spacer oligonucleotide, and then adding 1 to 6 nucleotides to the 3'-terminus thereof.
(5) An RNA capable of suppressing the expression of KLF5 gene, which is selected from the group consisting of the following (a) to (c):

(a) a double-stranded RNA having a strand of a sequence shown in any one of SEQ ID NOS: 2 to 16 and a strand of a sequence complementary to the sequence, in which 2 to 4 uridylic acids or deoxythymidylic acids are added to the 3'-terminus of each of the strands;
(b) an RNA forming a hairpin structure, which is obtained by ligating an RNA having a sequence shown in any one of SEQ ID NOS: 2 to 16 to an RNA having a sequence complementary to the sequence via a spacer RNA that has 2 uridylic acids at the 5'-terminus thereof, and then adding 2 to 4 uridylic acids to the 3'-terminus thereof; and
(c) a double-stranded RNA consisting of a strand of a sequence shown in any one of SEQ ID NOS: 2 to 11 and a strand of a sequence complementary to the sequence, in which 2 uridylic acids are added to the 3'-terminus of each of the strands.

(6) A vector, which allows the RNA according to any one of (1) to (5) to be expressed.
(7) A method of suppressing the expression of KLF5 gene in cells by transfecting the RNA according to any one of (1) to (5) above or the vector according to (6) into the cells.
(8) A method of suppressing the expression of a gene whose transcription is activated by KLF5 in cells by transfecting the RNA according to any one of (1) to (5) or the vector according to (6) into the cells.

(9) The method according to (8), wherein the gene whose transcription is activated by KLF5 is platelet-derived growth factor A chain gene or a smooth muscle myosin heavy chain SMemb gene.
(10) A pharmaceutical composition, which comprises, as an active ingredient, the RNA according to any one of (1) to (5) or the vector according to (6).
(11) A pharmaceutical composition for inhibiting angiogenesis, which comprises, as an active ingredient, the RNA according to any one of (1) to (5) or the vector according to (6).
(12) A therapeutic or preventive agent for cardiovascular disease or cancer, which comprises, as an active ingredient, the RNA according to any one of (1) to (5) or the vector according to (6).
(13) The therapeutic or preventive agent according to (12) above, wherein the cardiovascular disease is arteriosclerosis, restenosis occurring after coronary intervention, or cardiac hypertrophy.

By using the RNA of the present invention, the expression of KLF5 gene and a gene whose transcription is activated by KLF5 can be suppressed. The expression of the KLF5 gene and the gene whose transcription is activated by KLF5 is suppressed by administration of the RNA of the present invention or a vector for expressing the above RNA, and as a result, the growth of smooth muscle or vascularization can be suppressed. Thus, the RNA of the present invention or the vector for expressing the above RNA can be used as an active ingredient of a therapeutic or preventive agent for cardiovascular diseases such as arteriosclerosis, restenosis occurring after coronary intervention or cardiac hypertrophy, or cancers.

1. RNA capable of suppressing the expression of KLF5 gene

The RNA of the present invention comprises a sequence consisting of 15 to 30, preferably 17 to 25, and more preferably 19 to 23 contiguous nucleotides of KLF5 mRNA (hereinafter referred to as sequence X) and a sequence complementary to the above sequence (hereinafter referred to as complementary sequence X'), and it is capable of suppressing the expression of KLF5 gene. Such RNA includes: (a) a double-stranded RNA which consists of a strand of sequence X (sense strand) and a strand of complementary sequence X' (antisense strand), in which 1 to 6, and preferably 2 to 4 nucleotides are added to the 3'-terminus of each of the strands (hereinafter, an RNA having this structure is referred to as a siRNA), and which is capable of suppressing the expression of KLF5 gene; and (b) an RNA forming a hairpin structure, which is obtained by ligating an RNA having sequence X to an RNA having complementary sequence X' via a spacer oligonucleotide, and then adding 1 to 6, and preferably 2 to 4 nucleotides to the 3'-terminus thereof (hereinafter, such RNA is referred to as a shRNA), and which is capable of suppressing the expression of KLF5 gene. Nucleotides added to such an RNA may be any one selected from guanine, adenine, cytosine, thymine, and uracil. Either RNA or DNA may be used as a nucleotide added to such an RNA. Uridylic acid (U) or deoxythymidylic acid (dT) is preferable. As a spacer oligonucleotide, an RNA consisting of 6 to 12 nucleotides is preferable. As a sequence at the 5'-terminus thereof, two nucleotides, UU, are preferable. An example of such a spacer oligonucleotide is an RNA consisting of the sequence UUCAAGAGA. Either one of two RNA portions that are ligated to each other via a spacer oligonucleotide may be on the 5'-terminal side.
As sequence X, any sequence may be used, as long as it consists of 15 to 30, preferably 17 to 25, and more preferably 19 to 23 contiguous nucleotides of KLF5 mRNA. A sequence consisting of 19 nucleotides designed by the method described in (1) below is most preferable. An RNA that has the above-described structure and is capable of suppressing the expression of KLF5 gene is included in the RNA of the present invention.
The RNA of the present invention can be obtained by transfecting an RNA having the aforementioned structure into cells in which KLF5 gene is expressed, measuring the expression of KLF5 gene, and selecting an RNA suppressing the expression of KLF5 gene.

(1) Design of sequence X

A sequence portion consisting of 21 nucleotides that begin with AA is extracted from the nucleotide sequence of KLF5 cDNA of an animal in which the gene expression is to be suppressed. The GC content of the extracted sequence is calculated, and several sequences having a GC content between 20% and 80%, preferably between 30% and 70%, and more preferably between 40% and 60%, are selected.
Such a sequence is preferably a sequence in a coding region, which is located 75 nucleotides or more downstream of a start codon. Information regarding the nucleotide sequence of KLF5 cDNA can be obtained from nucleotide sequence database such as GenBank. For example, with regard to sequence information, the sequence of mouse KLF5 cDNA can be obtained from GenBank Accession No. NM_009769 (SEQ ID NO: 49), and the sequence of human KLF5 cDNA can be obtained from GenBank Accession No. AF287272 (SEQ ID NO: 50).
AA at the 5'-terminus is eliminated from the selected sequence, and T is then substituted with U in the sequence. The thus obtained sequence consisting of 19 nucleotides is defined as sequence X.

(2) Preparation of an RNA of the present invention

RNA can be prepared as follows based on sequence X selected in (1) above. A case where UU or dTdT are used as oligonucleotides to be added will be described below. However, RNA can also be prepared in the case that other nucleotides are used.

(a) Case of siRNA

An RNA having a sequence obtained by adding UU or dTdT to the 3'-terminus of sequence X, and an RNA having a sequence obtained by adding UU or dTdT to the 3'-terminus of complementary sequence X', are prepared. Such two RNA portions can be prepared by chemical synthesis or *in vitro* transcription. Chemical synthesis can be carried out using a DNA synthesizer. Otherwise, it is also possible to ask some manufacturers such as Ambion, Japan Bio Services Co., Ltd., or QIAGEN, to carry out such chemical synthesis. The thus chemically synthesized two RNA portions comprising sequences complementary to each other are annealed, so as to prepare a double-stranded RNA consisting of a strand of a sequence X and a strand of a complementary sequence X', in which UU or dTdT are added to the 3'-terminus of each of the strands. Annealing can be carried out by heating two RNA portions in a suitable buffer at a temperature between 90°C and 95°C for 1 to 5 minutes, and then cooling them to room temperature over 45 to 60 minutes.

RNA can be prepared via *in vitro* transcription as follows. First, the following DNA portions are prepared: (i) a DNA having the promoter sequence of T7 RNA polymerase (T7 primer); (ii) a DNA having a sequence obtained by substituting U with T in complementary sequence X', adding AA at the 5'-terminus thereof, and further adding to the 3'-terminus thereof a sequence complementary to 8 nucleotides of the 3'-terminus of the T7 primer; and (iii) a DNA having a sequence obtained by substituting U with T in sequence X, adding AA at the 5'-terminus thereof, and further adding to the 3'-terminus thereof a sequence complementary to 8 nucleotides of the 3'-terminus of the T7 primer.

The T7 primer and the DNA of (ii) are annealed, and thereafter, they are converted to double-stranded DNA by a DNA polymerase reaction. Using the obtained double-stranded DNA as a template, an *in vitro* transcription reaction is carried out using T7 RNA polymerase, so as to synthesize an RNA having a sequence obtained by adding UU to the 3'-terminus of sequence X and adding a leader sequence to the 5'-terminus thereof. Likewise, the same reaction as mentioned above is carried out using the T7 primer and the DNA of (iii), so as to synthesize an RNA having a sequence obtained by adding UU to the 3'-terminus of complementary sequence X' and adding a leader sequence to the 5'-terminus thereof.

The two reaction solutions are mixed, and such an *in vitro* transcription reaction is further continued, so that the two RNA portions having sequences complementary to each other are annealed. Thereafter, the double-stranded DNA used as a template and the leader sequence at the 5'-terminus of each RNA strand are digested with deoxyribonuclease and single-stranded RNA-specific ribonuclease, and then eliminated. The UU portion at the 3'-terminus of each RNA strand remains, without being digested.

The aforementioned reaction can be carried out using a kit such as Silencer • siRNA Construction Kit (manufactured by Ambion). DNA to be annealed with the T7 primer can be chemically synthesized using a DNA synthesizer. Moreover, it is also possible to ask some manufacturers such as Ambion, Japan Bio Services Co., Ltd., Hokkaido System Science Co., Ltd., or QIAGEN, to carry out such chemical synthesis.

(b) Case of shRNA

An RNA forming a hairpin structure obtained by ligating an RNA having sequence X to an RNA having complementary sequence X' via a spacer oligonucleotide, and then adding 1 to 6, and preferably 2 to 4 nucleotides, to the 3'-terminus thereof, can be prepared by chemical synthesis using a DNA synthesizer. In addition, an siRNA expression vector described in Section 2 later is transfected into cells, so as to synthesize shRNA in the cells. This shRNA is converted to siRNA in the cells. When a vector is transfected into cells and such shRNA is synthesized therein, isolation of a shRNA and transfection thereof into cells described in (3) below are unnecessary. The expression of KLF5 gene may only be analyzed in the cells transfected with the vector.

(3) Suppression of the expression of KLF5 gene

The siRNA or shRNA prepared in (2) above is transfected into a cell line that expresses KLF5 gene. As a cell line, the cells of the same animal species as the KLF5 cDNA used as a base of the design of sequence X described in (1) above are used. Examples of a cell line that expresses KLF5 gene may include cell lines derived from smooth muscle, fibroblasts or vascular endothelial cells, such as the fetal mouse fibroblast cell line C3H/10T1/2 (ATCC No. CCL-226) or human umbilical cord vascular endothelial cells. Transfection of the RNA can be carried out using reagents for transfection into animal cells, such as Polyfect Transfection Reagent (manufactured by QIAGEN), TransMessenger Transfection Reagent, Oligofectamine Reagent (manufactured by Invitrogen), or Lipofectamine 2000 (manufactured by Invitrogen). These reagents are mixed with the RNA to form a complex, and the complex is then added to cells.

The expression of KLF5 gene in cells, which were transfected with the RNA of the present invention or an siRNA expression vector described later in Section 2, can be analyzed by RT-PCR. Total RNA is prepared from cells transfected with the RNA or the siRNA expression vector, and also from cells which were not transfected with the RNA or the siRNA expression vector. Thereafter, cDNA is synthesized from the RNA. Using the synthesized cDNA as a template, PCR is carried out with primers specific to KLF5 gene. The amount of an amplified product derived from KLF5 cDNA is quantified by agarose gel electrophoresis, thereby measuring the expression level of KLF5 gene. An RNA that was transfected into cells in which the expression level of the KLF5 gene is lower than the expression level of the KLF5 gene in cells which were not transfected with the RNA or the siRNA expression vector, is selected as an RNA capable of suppressing the expression of KLF5 gene.

An example of the thus selected RNA capable of suppressing the expression of KLF5 gene is a double-stranded RNA consisting of a strand of a sequence shown in any one of SEQ ID NOS: 2 to 11 and a strand of a sequence complementary to the above sequence, in which two uridylic acids are added to the 3'-terminus of each of the strands. This RNA is designed based on the sequence of mouse cDNA, and it suppresses the expression of mouse KLF5 gene. Among such sequences, sequences shown in SEQ ID NOS: 4, 8, and 10, are shared by mouse KLF5 mRNA and human KLF5 mRNA. Thus, a double-stranded RNA consisting of a strand of a sequence shown in any one of SEQ ID NOS: 4, 8, and 10, and a strand of a sequence complementary to the above sequence, in which two uridylic acids are added to the 3'-terminus of each of the strands, is capable of suppressing not only the mouse KLF5 gene but also the human KLF5 gene.

The KLF5 cDNA of a certain animal species "A" used as a base for the design of sequence X described in (1) above is aligned with the KLF5 cDNA of a different animal species "B" based on the sequence homology, so as to obtain sequence Y of the animal species "B" that corresponds to sequence X selected in the animal species "A". When an RNA capable of suppressing the expression of KLF5 gene of the animal species "A" is obtained by the aforementioned method, an RNA obtained by substituting sequence X region and complementary sequence X' region with sequence Y and complementary sequence Y' in the RNA, respectively, is considered to be capable of suppressing KLF5 gene of the animal species "B".

For example, a double-stranded RNA consisting of a strand of a sequence shown in any one of SEQ ID NOS: 2, 3, 7, 9, and 11, based on the sequence of mouse KLF5 cDNA, and a strand of a sequence complementary to the above sequence, in which two uridylic acids are added to the 3'-terminus of each of the strands, is capable of suppressing the expression of mouse KLF5 gene. Accordingly, a double-stranded RNA consisting of a strand of a sequence shown in any one of SEQ ID NOS: 12 to 16, which are the corresponding sequences of human KLF5 cDNA, and a strand of a sequence complementary to the above sequence, in which two uridylic acids are added to the 3'-terminus of each of the strands, is considered to be capable of suppressing the expression of the human KLF5 gene.

2. Vector for expressing RNA capable of suppressing the expression of KLF5 gene

(1) Plasmid vector

A plasmid vector for expressing an RNA capable of suppressing the expression of KLF5 gene is transfected into cultured cells or cells in a living body, so that the above RNA can be generated in the cells, thereby suppressing the expression of KLF5 gene in the transfected cells. This vector can be produced by inserting downstream of the promoter of an siRNA expression vector such as a plasmid vector used for animal cells comprising an RNA polymerase III promoter such as U6 promoter or H1 promoter, a DNA obtained by ligating sequence X selected in Section 1 to complementary sequence X' thereof (wherein, in each sequence, U is substituted with T) via a spacer sequence having TT at the 5'-terminus thereof, which comprises a sequence portion consisting of 4 to 6 Ts acting as an RNA polymerase III terminator at the 3'-terminus thereof (hereinafter referred to as DNA used for KLF5 siRNA). As a spacer sequence, a sequence consisting of 6 to 12 nucleotides and having TT at the 5'-terminus thereof is preferable. An example of such a sequence is TTCAAGAGA. Either one of sequence X and complementary sequence X' may be on the 5'-terminal side. Examples of an siRNA expression vector may include pSilencer 1.0-U6 (manufactured by Ambion), pSilencer 3.0 (manufactured by Ambion), pSUPER (manufactured by OligoEngine), and pSIREN-DNR (manufactured by BD Biosciences Clontech).

In cells transfected with a recombinant vector constructed by insertion of the aforementioned DNA used for KLF5 siRNA, the shRNA described in Section 1. (1) is synthesized by an RNA polymerase III reaction from U6 promoter. Thereafter, this shRNA is cleaved in the cells and then converted to a siRNA. Such a recombinant vector can be transfected into cells by the calcium phosphate method (Japanese Published Unexamined Patent Application no. 227075/90), the lipofection method (Proc. Natl. Acad. Sci. USA, 84, 7413-7417, 1987), or the like, as with common transfection of a vector into animal cells.

(2) Viral vector

As an siRNA expression vector, a siRNA expression vector utilizing viral vectors such as a retrovirus vector, lentivirus vector, or adenovirus vector, can be used. Examples of an siRNA expression vector utilizing such viral vectors may include pSUPER.retro (manufactured by OligoEngine), pSIREN-RetroQ (BD Biosciences • Clontech), and a vector described in the publication (Proc. Natl. Acad. Sci USA, 100, 1844-1848, 2003; Nat. Genet., 33, 401-406, 2003).

The same DNA used for KLF5 siRNA as mentioned above is inserted into an siRNA expression vector utilizing a viral vector, so as to prepare a recombinant vector. Thereafter, the obtained vector is transfected into packaging cells which are selected based on the utilized viral vector, thereby producing a recombinant virus comprising the above recombinant vector. Such a recombinant vector can be transfected into packaging cells by the calcium phosphate method or the lipofection method, as described above. The obtained recombinant virus is allowed to contact with cells, and the cells are thereby infected therewith, so as to transfect a recombinant vector into the cells. As a result, the shRNA described in Section 1. (1) is synthesized, and the synthesized shRNA is then cleaved in the cells, so that the shRNA can be converted to a siRNA capable of suppressing the expression of KLF5 gene.

3. Use of RNA capable of suppressing the expression of KLF5 gene

(1) Suppression of the expression of gene whose transcription is activated by KLF5

KLF5 acts as a transcription factor and activates the expression of various genes. When the expression of KLF5 gene is suppressed by an RNA capable of suppressing it, the expression of genes whose transcription is activated by KLF5 can also be suppressed. Examples of such a gene whose transcription is activated by KLF5 may include the genes such as SMemb, PDGF-A, TGF-β, a VEGF receptor, PAI-1, and Egr-1.

(2) Analysis of functions of KLF5

An RNA capable of suppressing the expression of KLF5 gene is allowed to act on various types of cells, and a change in the characteristics of the cells or a change in the expression level of each gene are then examined, so as to analyze the functions of KLF5 in various types of cells. In addition, the above RNA is capable of suppressing the expression of KLF5 gene in animals that are at various development stages ranging from a fetus to an adult, and thus it becomes possible to clarify the functions of KLF5, which cannot be clarified only by the analysis of hetero-knockout mice.

4. Pharmaceutical composition comprising, as active ingredient, RNA or vector of the present invention

The expression of KLF5 and the genes whose transcription is activated by KLF5 is suppressed by administration of an RNA that is specifically capable of suppressing the expression of KLF5 gene of the present invention or a vector for expressing the above RNA, so as to inhibit the growth of smooth muscle or the angiogenesis. Hence, the above RNA or vector can be used to treat or prevent cardiovascular diseases such as arteriosclerosis, restenosis occurring after coronary intervention or cardiac hypertrophy, or cancers.

When the RNA of the present invention or a vector for expressing the above RNA is used as a medicament, it can be administered singly. In general, however, it is preferable that such an RNA or a vector be mixed with pharmacologically acceptable additives (for example, a carrier, an excipient, a diluent, etc.), stabilizers, or pharmaceutically necessary components, and that it be provided in the form of a pharmaceutical preparation produced by any given method well known in the technical field of pharmaceutics. In the case of a viral vector, it is preferably administered in the form of a recombinant virus.

It is preferable that an administration route that is most effective for treatment be used. Examples of an administration route used herein may include: parenteral administration routes such as intraoral administration, tracheobronchial administration, intrarectal administration, subcutaneous administration, intramuscular administration, or intravenous administration; and an oral administration route. Preferred administration routes include intravenous administration and intramuscular administration. An example of a pharmaceutical preparation suitable for intravenous administration or intramuscular administration is an injection.

When the RNA of the present invention or a vector for expressing the above RNA is molded in the form of an injection, examples of a carrier used herein may include: diluents such as water, ethyl alcohol, macrogol, propylene glycol, citric acid, acetic acid, phosphoric acid, lactic acid, sodium lactate, sulfuric acid, or sodium hydroxide; pH adjusters and buffers such as sodium citrate, sodium acetate, or sodium phosphate; and stabilizers such as sodium pyrosulfite, ethylenediaminetetraacetic acid, thioglycolic acid, or thiolactic acid. In such a case, common salt, glucose, mannitol, or glycerin may be contained in the pharmaceutical preparation, at an amount sufficient for preparation of an isotonic solution. Examples of a stabilizer may include: monosaccharides such as glucose; disaccharides such as saccharose or maltose; sugar alcohols such as mannitol or sorbitol; neutral salts such as sodium chloride; amino acids such as glycine; nonionic surfactants such as polyethylene glycol, a polyoxyethylene-polyoxypropylene copolymer (Pluronic), or polyoxyethylene sorbitan fatty acid ester (Tween); and human albumin. In addition, in order to promote incorporation of the RNA of the present invention or a vector expressing the above RNA into cells, a liposome comprising the above RNA or vector may be prepared and used.

BRIEF DESCRIPTION OF THE DRAWINGS

Figure 1 shows suppression of the expression of KLF5 gene by KLF5 gene-specific siRNA. From the left, the figure shows a 100-bp marker, and the results obtained by measurement by PCR on cells which were not transfected with a siRNA, and cells which were transfected with each of SEAP-siRNA, siRNA No. 2, siRNA No. 3, siRNA No. 4, siRNA No. 5, and siRNA No. 6. KLF5 represents the position of an amplified product derived from KLF5 mRNA, and 18S represents the position of an amplified product derived from 18S rRNA.

Figure 2 shows suppression of the expression of KLF5 gene by KLF5 gene-specific siRNA. From the left, the figure shows a 100-bp marker, and the results obtained by measurement by PCR on cells which were not transfected with a siRNA, and cells which were transfected with each of SEAP-siRNA, siRNA No. 7, siRNA No. 8, siRNA No. 9, siRNA No. 10, siRNA No. 11, siRNA No. 4, and siRNA No. 1. KLF5 represents the position of an amplified product derived from KLF5 mRNA, and 18S represents the position of an amplified product derived from 18S rRNA.

Figure 3 shows suppression of the expression of PDGF-A gene by KLF5 gene-specific siRNA. From the left, the figure shows a 100-bp marker, and the results obtained by measurement by PCR on cells which were not transfected with a siRNA, and cells which were transfected with each of SEAP-siRNA, siRNA No. 7, siRNA No. 8, siRNA No. 9, siRNA No. 10, siRNA No. 11, siRNA No. 4, and siRNA No. 1. PDGF-A represents the position of an amplified product derived from PDGF-A mRNA, and 18S represents the position of an amplified product derived from 18S rRNA.

Figure 4 shows suppression of the expression of SMemb gene by KLF5 gene-specific siRNA. From the left, the figure shows a 100-bp marker, and the results obtained by measurement by PCR on cells which were not transfected with a siRNA, and cells which were transfected with each of SEAP-siRNA, siRNA No. 7, siRNA No. 8, siRNA No. 9, siRNA No. 10, siRNA No. 11, siRNA No. 4, and siRNA No. 1. SMemb represents the position of an amplified product derived from SMemb mRNA, and 18S represents the position of an amplified product derived from 18S rRNA.

Figure 5 shows that KLF5 gene-specific siRNA does not suppress the expression of SRF gene. From the left, the figure shows the results obtained by measurement by PCR on cells which were not transfected with a siRNA, and cells which were transfected with each of SEAP-siRNA, siRNA No. 1, siRNA No. 4, siRNA No. 7, siRNA No. 9, and siRNA No. 10. SRF represents the position of an amplified product derived from SRF mRNA, and 18S represents the position of an amplified product derived from 18S rRNA.

Figure 6 shows suppression of the expression of human KLF5 gene by siRNA No. 4. From the left, the figure shows a 100-bp marker, and the results obtained by measurement by PCR on cells which were not transfected with a siRNA, and cells which were transfected with either SEAP-siRNA or siRNA No. 4. KLF represents the position of an amplified product derived from KLF5 mRNA, and 18S represents the position of an amplified product derived from 18S rRNA.

Figure 7 shows inhibition of the migration of vascular endothelial cells by siRNA No. 4. The horizontal axis represents time (hours), and the longitudinal axis represents the number of cells migrated. ● represents the results regarding cells transfected with siRNA No. 4, and ■ represents the results regarding cells transfected with SEAP-siRNA. The error bar represents a standard deviation of 4 cases.

Figure 8 shows the antitumor effect of siRNA No. 4. The horizontal axis represents time (number of days), and the longitudinal axis represents tumor volume ($mm^3$). ● represents the tumor volume of a mouse to which KLF5 siRNA No. 4 has been administered, and ■ represents the tumor volume of a mouse to which SEAP-siRNA has been administered.

BEST MODE FOR CARRYING OUT THE INVENTION

The present invention will be more specifically described in the following examples. However, the present invention is not limited by these examples.

Example 1 Suppression of the expression of KLF5 gene by siRNA

(1) Preparation of siRNA

As the sequence of siRNA which can suppress expression of KLF5 gene, from the sequence of mouse KLF5 cDNA (GenBank Registration No. NM_009769; SEQ ID NO: 49), 11 sequence portions that satisfy the following 2 requirements were selected: (a) a sequence consisting of 21 nucleotides that begin with AA; and (b) a GC content between 20% and 80%. Such sequence portions were preferably selected from sequence portions, which are located in a coding region (the sequence portion at nucleotides 167-1507 of SEQ ID NO: 49) and further located 75 nucleotides or more downstream of a start codon (the sequence portion at nucleotides 167-169 of SEQ ID NO: 49), and which have a GC content between 40% and 60%. The positions of the selected sequences in SEQ ID NO: 49 and GC contents thereof are shown in Table 1. Sequences obtained by substituting T with U in 19 nucleotides excluding AA at the 5'-terminus thereof in the selected sequences were shown in SEQ ID NOS: 1 to 11.

Table 1

| Sequence selected | Sequence position | GC content | Produced RNA sequence | SEQ ID No. | siRNA No. |
|---|---|---|---|---|---|
| AACATGAACGTCTTCCTCCCT | 537-556 | 48% (10/21) | CAUGAACGUCUUCCUCCCUTT | 17 | No. 1 |
| | | | AGGGAGGAAGACGUUCAUGTT | 18 | |
| AAATTTACCTGCCACTCTGCC | 1156-1176 | 48% (10/21) | AUUUACCUGCCACUCUGCCUU | 19 | No. 2 |
| | | | GGCAGAGUGGCAGGUAAAUUU | 20 | |
| AAGGAGTAACCCGGATCTGGA | 1216-1236 | 52% (11/21) | GGAGUAACCCGGAUCUGGAUU | 21 | No. 3 |
| | | | UCCAGAUCCGGGUUACUCCUU | 22 | |
| AAAAGCTCACCTGAGGACTCA | 1303-1323 | 48% (10/21) | AAGCUCACCUGAGGACUCAUU | 23 | No. 4 |
| | | | UGAGUCCUCAGGUGAGCUUUU | 24 | |
| AATCCCCAGACCGTCCATGCC | 151-171 | 62% (13/21) | UCCCCAGACCGUCCAUGCCUU | 25 | No. 5 |
| | | | GGCAUGGACGGUCUGGGGGUU | 26 | |
| AACGCTGCGCCCACCCGCCTG | 1515-1535 | 76% (16/21) | CGCUGCGCCCACCCGCCUGUU | 27 | No. 6 |
| | | | CAGGCGGGUGGGCGCAGCGUU | 28 | |
| AAATGGAGAAGTATCTGACCC | 405-425 | 43% (9/21) | AUGGAGAAGUAUCUGACCCUU | 29 | No. 7 |
| | | | GGGUCAGAUACUUCUCCAUUU | 30 | |
| AAAGTATAGACGAGACAGTGC | 463-483 | 43% (9/21) | AGUAUAGACGAGACAGUGCUU | 31 | No. 8 |
| | | | GCACUGUCUCGUCUAUACUUU | 32 | |
| AAACCAGACGGCAGTAATGGA | 874-894 | 48% (10/21) | ACCAGACGGCAGUAAUGGAUU | 33 | No. 9 |
| | | | UCCAUUACUGCCGUCUGGCUU | 34 | |
| AAGCTCAGAGCCTGGAAGTCC | 2048-2068 | 57% (12/21) | GCUCAGAGCCUGGAAGUCCUU | 35 | No. 10 |
| | | | GGACUUCCAGGCUCUGAGCUU | 36 | |
| AAGCCGTTCCAGTGCATGGTG | 1424-1444 | 57% (12/21) | GCCGUUCCAGUGCAUGGUGUU | 37 | No. 11 |
| | | | CACCAUGCACUGGAACGGCUU | 38 | |

11 types of double-stranded RNA (hereinafter referred to as siRNA Nos. 1 to 11), which have a sequence shown in any one of SEQ ID NOS: 1 to 11 and a sequence complementary thereto, wherein UU or dTdT are added to the 3'-terminus of each of sequences, were prepared as follows. The sequences of the sense strand and antisense strand of each of siRNA Nos. 1 to 11 are shown in Table 1 (SEQ ID NOS: 17 to 38). siRNA No. 1 was prepared as follows. Namely, chemical synthesis of two RNAs shown in SEQ ID NOS: 17 and 18 was carried out by Japan Bio Services, Co., Ltd., and the thus obtained RNAs were annealed with each other. SiRNA Nos. 2 to 11 were prepared by *in vitro* transcription using Silencer™ siRNA Construction Kit (manufactured by Ambion). DNA used to produce a template used for the *in vitro* transcription was prepared by Hokkaido System Science Co., Ltd. Moreover, siRNA having sequences shown in SEQ ID NOS: 39 and 40, which is capable of suppressing the expression of secretory alkaline phosphatase (SEAP) gene (hereinafter referred to as SEAP-siRNA), was prepared by *in vitro* transcription using Silencer siRNA Construction Kit, based on the publications (Nat. Genet., 32, 107-108, 2002; and US patent Application Laid-Open No. 2002/0132788). This siRNA was used as a control siRNA.

(2) Suppression of the expression of KLF5 gene by siRNA

The fetal mouse fibroblast cell line C3H/10T1/2 (obtained from American Type Culture Collection (ATCC); ATCC No. CCL-226) was inoculated in a 6-well plate (manufactured by Corning), resulting in a concentration of 4 x $10^5$ cells per well. Thereafter, 10 μl of a reagent for transfection into cells, Polyfect (manufactured by QIAGEN), was added to each of 1.5 μg of siRNA No. 2, No. 3, No. 4, No. 5 and No. 6, and SEAP-siRNA, and then mixed. Each of the obtained mixtures was retained at room temperature for 5 to 10 minutes, and it was then added to each well. The cells were incubated in the presence of 5% $CO_2$ at 37°C for 48 to 72 hours, so that each siRNA was transfected into the cells.

Suppression of the expression of KLF5 gene by the siRNA was confirmed by RT-PCR as shown below. After the incubation, RNA was isolated from the recovered cells, using a cell lysate-homogenizing kit QIAshredder (manufactured by QIAGEN) and a total RNA purification kit RNeasy (manufactured by QIAGEN). The isolated RNA was dissolved in 30 to 50 μl of water for injection (Otsuka distilled water, manufactured by Otsuka Pharmaceutical Co., Ltd.), and cDNA was then synthesized by a reverse transcription reaction. The reverse transcription reaction was carried out at 42°C for 1.5 hours, using a reaction solution prepared by mixing the aforementioned RNA solution (containing 1.0 μg of RNA) with a solution containing 2.5 μl of 5 x buffer, 2.0 μl of 0.1 mol/L dithiothreitol (DTT), 1.0 μl of 20 mmol/L dNTP (manufactured by Roche), 2.0 μl of a 50 μmol/L random primer (manufactured by Takara Shuzo Co., Ltd.), 1.0 gl of nuclease inhibitor SUPERase•In (manufactured by Ambion), and 1.0 μl of PowerScript reverse transcriptase (manufactured by Clontech), and then adding the water for injection thereto to a total amount of 18 μl. As such 5 x buffer and DTT, those attached with the PowerScript reverse transcriptase were used.

Two DNAs each having the sequence shown in SEQ ID NO: 41 or 42 were chemically synthesized. These DNAs were used as mouse KLF5 gene-specific forward primer and reverse primer. By carrying out PCR using these primers, a 161-bp fragment corresponding to a sequence portion from nucleotides 1268 to 1428 of SEQ ID NO: 49 is amplified from KLF5 cDNA.

There was prepared 25 μl of a PCR reaction solution consisting of 2.5 μl of 10 x PCR buffer, 2.0 μl of 2.5 mmol/L dNTP (manufactured by Roche), 2.0 μl of a 5 μmol/L forward primer, 2.0 μl of a 5 μmol/L reverse primer, 0.125 μl of HotStarTaq DNA polymerase (manufactured by QIAGEN; 5 units/μL), 2 μl of a 18S rRNA-specific primer (QuantumRNA Classic 18S Internal Standard; manufactured by Ambion), 13.375 μl of the water for injection, and 1.0 μl of cDNA. The prepared reaction solution was retained at 95°C for 15 minutes. Thereafter, a reaction consisting of heat denaturation at 94°C for 30 seconds, annealing at 53°C for 30 seconds, and elongation at 72°C for 40 seconds, was defined as 1 cycle, and PCR of 28 cycles was carried out. Thereafter, the reaction product was retained at 72°C for 10 minutes. As a 10 x PCR buffer, a buffer attached with the HotStarTaq DNA polymerase was used. After completion of the reaction, the obtained reaction solution was subjected to 0.8% agarose gel electrophoresis, so as to detect an amplified product (161 bp) derived from KLF5 mRNA. The amount of the obtained amplified product was compared with the amount of an amplified product in cells which were not transfected with a siRNA. An amplified product (488 bp) derived from 18S rRNA was used as an internal standard. As shown in Figure 1, SEAP-siRNA used as a control did not suppress the expression of KLF5 gene, whereas it was confirmed that KLF5 gene-specific siRNA No. 2, No. 3, No. 4, No. 5, and No. 6, suppressed the expression of KLF5 gene. Among them, siRNA No. 3 and siRNA No. 4 strongly suppressed the expression of KLF5 gene.

Using siRNA No. 1, No. 4, No. 7, No. 8, No. 9, No. 10, and No. 11, as KLF5 gene-specific siRNA, such siRNA was transfected into C3H/10T1/2 cells and the expression of KLF5 gene was then analyzed by RT-PCR, as described above. As shown in Figure 2, SEAP-siRNA used as a control did not suppress the expression of KLF5 gene, whereas it was confirmed that KLF5 gene-specific siRNA No. 4, No.7, No. 8, No. 9, No. 10, and No. 11, suppressed the expression of KLF5 gene. Among them, siRNA No. 4, siRNA No. 7, siRNA No. 9, and siRNA No. 10, strongly suppressed the expression of KLF5 gene. Although siRNA No. 1 is specific to the KLF5 gene, it did not suppress the expression thereof.

Example 2 Suppression of the expression of genes whose transcription is activated by KLF5, by KLF5 gene-specific siRNA

(1) Suppression of the expression of PDGF-A gene

As KLF5 gene-specific siRNA, each of siRNA No. 1, No. 4, No. 7, No. 8, No. 9, No. 10, and No. 11, was transfected into C3H/10T1/2 cells. Thereafter, the expression of PDGF-A gene whose transcription is activated by KLF5 was analyzed by RT-PCR.

Each siRNA was transfected into C3H/10T1/2 cells and cDNA was then prepared in the same manner as in Example 1(2). Two DNAs each having the sequence shown in SEQ ID NO: 43 or 44 were chemically synthesized. These DNAs were used as PDGF-A gene-specific forward primer and reverse primer. By carrying out PCR using these primers, a 403-bp fragment is amplified from PDGF-A cDNA. The PDGF-A gene-specific forward primer and reverse primer were used instead of the KLF5 gene-specific forward primer and reverse primer, and the expression of PDGF-A gene was analyzed in the same manner as in the analysis of the expression of KLF5 gene of Example 1(2). The PCR reaction solution was retained at 95°C for 15 minutes. Thereafter, a reaction consisting of heat denaturation at 94°C for 30 seconds, annealing at 53°C for 30 seconds, and elongation at 72°C for 40 seconds, was defined as 1 cycle, and PCR of 26 cycles was carried out. Thereafter, the reaction product was retained at 72°C for 10 minutes. After completion of the reaction, the obtained reaction solution was subjected to 1% agarose gel electrophoresis.

As shown in Figure 3, SEAP-siRNA used as a control did not suppress the expression of PDGF-A gene, whereas it was confirmed that KLF5 gene-specific siRNA No. 4, No.7, No. 8, No. 9, No. 10, and No. 11 suppressed also the expression of PDGF-A gene whose transcription is activated by KLF5. Among them, siRNA No. 4, siRNA No. 7, siRNA No. 9, and siRNA No. 10, strongly suppressed the expression of PDGF-A gene. Although siRNA No. 1 is specific to the KLF5 gene, it did not suppress the expression thereof.

(2) Suppression of the expression of SMemb gene

As KLF5 gene-specific siRNA, each of siRNA No. 1, No. 4, No. 7, No. 8, No. 9, No. 10, and No. 11, was transfected into C3H/10T1/2 cells. Thereafter, the expression of SMemb gene whose transcription is activated by KLF5 was analyzed by RT-PCR.

Each siRNA was transfected into C3H/10T1/2 cells and cDNA was then prepared in the same manner as in Example 1(2). Two DNAs each having the sequence shown in SEQ ID NO: 45 or 46 were chemically synthesized. These DNAs were used as SMemb gene-specific forward primer and reverse primer. By carrying out PCR using these primers, a 235-bp fragment is amplified from SMemb cDNA. The SMemb gene-specific forward primer and reverse primer were used instead of the KLF5 gene-specific forward primer and reverse primer, and the expression of SMemb gene was analyzed in the same manner as in the analysis of the expression of KLF5 gene of Example 1(2). The PCR reaction solution was retained at 95°C for 15 minutes. Thereafter, a reaction consisting of heat denaturation at 94°C for 30 seconds, annealing at 53°C for 30 seconds, and elongation at 72°C for 40 seconds, was defined as 1 cycle, and PCR of 26 cycles was carried out. Thereafter, the reaction product was retained at 72°C for 10 minutes. After completion of the reaction, the obtained reaction solution was subjected to 1% agarose gel electrophoresis.

As shown in Figure 4, SEAP-siRNA used as a control did not suppress the expression of SMemb gene, whereas it was confirmed that KLF5 gene-specific siRNA No. 4, No.7, No. 8, No. 9, No. 10, and No. 11 suppressed also the expression of SMemb gene whose transcription is activated by KLF5. Among them, siRNA No. 4, siRNA No. 7, siRNA No. 9, and siRNA No. 10, strongly suppressed the expression of SMemb gene. Although siRNA No. 1 is specific to the KLF5 gene, it did not suppress the expression thereof.

(3) Specificity of suppression of gene expression by KLF5 gene-specific siRNA

KLF5 gene-specific siRNA was transfected into C3H/10T1/2 cells, and the expression of serum response factor (SRF) gene was then analyzed by RT-PCR, so as to verify that suppression of gene expression by KLF5 gene-specific siRNA is specific to the KLF5 gene or genes whose transcription is activated by KLF5. The SRF gene is a transcription factor gene that is highly expressed in smooth muscle cells, and it is not a gene whose transcription is activated by KLF5.

As KLF5 gene-specific siRNA, each of siRNA No. 1, No. 4, No. 7, No. 9, and No. 10 were used. Each siRNA was transfected into C3H/10T1/2 cells and the gene expression was then analyzed by RT-PCR in the same manner as in Example 1(2). Two DNAs each having the sequence shown in SEQ ID NO: 47 or 48 were chemically synthesized. These DNAs were used as SRF gene-specific forward primer and reverse primer. By carrying out PCR using these primers, a 519-bp fragment is amplified from SRF cDNA. The SRF gene-specific forward primer and reverse primer were used instead of the KLF5 gene-specific forward primer and reverse primer, and the expression of SRF gene was analyzed in the same manner as in the analysis of the expression of KLF5 gene of Example 1(2). The PCR reaction solution was retained at 95°C for 15 minutes. Thereafter, a reaction consisting of heat denaturation at 94°C for 30 seconds, annealing at 53°C for 30 seconds, and elongation at 72°C for 40 seconds, was defined as 1 cycle, and PCR of 26 cycles was carried out. Thereafter, the reaction product was retained at 72°C for 10 minutes. After completion of the reaction, the obtained reaction solution was subjected to 1.2% agarose gel electrophoresis.

As shown in Figure 5, as in the case of SEAP-siRNA used as a control, all of KLF5 gene-specific siRNA No. 1, No. 4, No. 7, No. 9, and No. 10 did not suppress the expression of SRF gene. Therefore, it became clear that KLF5 gene-specific siRNA does not suppress the expression of all such genes non-specifically, but that it specifically suppresses the expression of KLF5 gene and genes whose transcription is activated by KLF.

Example 3 Suppression of the expression of human KLF5 gene by siRNA

The siRNA No. 4 prepared in Example 1 is siRNA produced based on the sequence (AAAAGCTCACCTGAGGACTCA) at nucleotides 1303 to 1323 of the nucleotide sequence (SEQ ID NO: 49) of mouse KLF5 cDNA. The siRNA No. 4 strongly suppressed the expression of mouse KLF5 gene in C3H/10T1/2 cells. However, this sequence, AAAAGCTCACCTGAGGACTCA, also exists at nucleotides 1481 to 1501 of the nucleotide sequence (SEQ ID NO: 50) of human KLF5 cDNA. Thus, it is anticipated that siRNA No. 4 inhibit not only the expression of mouse KLF5 gene but also that of human KLF5 gene. As described below, it was confirmed that siRNA No. 4 strongly suppressed the expression of human KLF5 gene.

Human umbilical cord venous endothelial cells (HUVEC; procured from Sanko Junyaku Co., Ltd.; Production No. CC-2517) were inoculated into a 6-cm dish (Corning), resulting in a concentration of approximately 3 x $10^5$ cells. 10 μl of a cell transfection reagent (Lipofectamine 2000, manufactured by Invitrogen) was added to each of 200 pmol siRNA No. 4 and SEAP-siRNA, and then mixed. The obtained mixture was retained at room temperature for 20 minutes, and the total amount thereof was then added to each dish. Thereafter, the cells were incubated in the presence of 5% $CO_2$ at 37°C for 24 hours, so as to transfect each siRNA into the cells.

Thereafter, RNA was isolated from the cells by the same method as described in Example 1(2), and suppression of the expression of human KLF5 gene was then examined by RT-PCR. DNAs each having the sequence shown in SEQ ID NO: 41 or 42 which were used in Example 1 were used as KLF gene-specific forward primer and reverse primer, respectively. By carrying out PCR using these primers, a 161-bp fragment corresponding to a sequence portion from nucleotides 1446 to 1606 of SEQ ID NO: 50 is amplified from human KLF5 cDNA. After completion of the reaction, the obtained reaction solution was subjected to 0.8% agarose gel electrophoresis, so as to detect an amplified product (161 bp) derived from KLF5 mRNA. The amount of the obtained amplified product was compared with the amount of an amplified product in cells which were not transfected with a siRNA. An amplified product (488 bp) derived from 18S rRNA was used as an internal standard. As shown in Figure 6, SEAP-siRNA used as a control did not suppress the expression of KLF5 gene, whereas KLF5 gene-specific siRNA No. 4 suppressed the expression of KLF5 gene in the human umbilical cord venous endothelial cells. Thus, it was confirmed that siRNA No. 4 could strongly suppress not only mouse KLF5 gene but also a human KLF5 gene.

With regard to siRNA Nos. 2 to 4 and 7 to 11 that suppressed the expression of a mouse KLF5 gene in Example 1, Table 2 shows: sequence portions consisting of 21 nucleotides on mouse KLF5 cDNA used as bases for the design of siRNA Nos. 2 to 4 and 7 to 11, and the positions thereof on SEQ ID NO: 49; sequence portions consisting of 21 nucleotides on human cDNA corresponding to the above mouse sequences, and the positions thereof on SEQ ID NO: 50; and SEQ ID NOS indicating RNA sequences obtained by eliminating AA at the 5'-terminus from the above human sequences. Since siRNA No. 5 and No. 6 were based on the sequence of a non-coding region, human sequences corresponding thereto were not indicated. It is considered that double-stranded RNA produced based on these human sequences also suppresses the expression of human KLF5 gene. With regard to siRNA Nos. 4, 8, and 10, human sequences corresponding thereto are completely the same as mouse sequences. Thus, it was considered that siRNA Nos. 8 and 10 suppress not only mouse KLF5 gene but also human KLF5 gene, as in the case of siRNA No. 4.

Table 2

| siRNA No. | Mouse KLF5 cDNA | | Human KLF5 cDNA | | |
|---|---|---|---|---|---|
| | Sequence | Position | Corresponding sequence | Position | SEQ ID No. |
| No. 2 | AAATTTACCTGCCACTCTGCC | 1156-1176 | AAATTTACCCACCACCCTGCC | 1334-1354 | 12 |
| No. 3 | AAGGAGTAACCCGGATCTGGA | 1216-1236 | AAGGAGTAACCCCGATTTGGA | 1394-1414 | 13 |
| No. 4 | AAAAGCTCACCTGAGGACTCA | 1303-1323 | AAAAGCTCACCTGAGGACTCA | 1481-1501 | 4 |
| No. 7 | AAATGGAGAAGTATCTGACCC | 405-425 | AAATGGAGAAGTATCTGACAC | 583-603 | 14 |
| No. 8 | AAAGTATAGACGAGACAGTGC | 463-483 | AAAGTATAGACGAGACAGTGC | 641-661 | 8 |
| No. 9 | AAACCAGACGGCAGTAATGGA | 874-894 | AAATCAGACAGCAGCAATGGA | 1040-1060 | 15 |
| No. 10 | AAGCTCAGAGCCTGGAAGTCC | 2048-2068 | AAGCTCAGAGCCTGGAAGTCC | 1226-1246 | 10 |
| No. 11 | AAGCCGTTCCAGTGCATGGTG | 1424-1444 | AAGCCCTTCCAGTGCGGGGTG | 1602-1622 | 16 |

Example 4 Inhibition of migration of vascular endothelial cells by siRNA capable of suppressing expression of KLF5 gene

Inhibition of the migration of vascular endothelial cells by siRNA No. 4 capable of suppressing the expression of KLF5 gene was examined by performing an *in vitro* cell migration test on the vascular endothelial cells, using the following micropore filter (J. Cell Biol., 147, 1073-1084, 1999; Becton, Dickinson and Company, Technical Bulletin, 429, 1998).

Human umbilical cord venous endothelial cells (HUVEC; obtained from Sanko Junyaku Co., Ltd.; Production No. CC-2517) were inoculated into a 6-cm dish (Corning), resulting in a concentration of approximately 3 x $10^5$ cells. 10 μl of a cell transfection reagent (Lipofectamine 2000, manufactured by Invitrogen) was added to each of 200 pmol siRNA No. 4 and SEAP-siRNA used as a control, and then mixed. The obtained mixture was incubated at room temperature for 20 minutes, and the total amount thereof was then added to each dish. Thereafter, the cells were incubated in the presence of 5% $CO_2$ at 37°C for 18 hours, so as to transfect each siRNA into the cells.

Thereafter, siRNA-transfected cells were washed, and the cells were then fluorescently labeled with 5 μg/ml fluorescent dye for staining living cells (Calcein AM, manufactured by Dojindo Laboratories). The thus obtained fluorescently labeled cells were peeled off with trypsin and then washed. Thereafter, the resultant cells were resuspended in a basal medium for vascular endothelial cells (EBM-2, manufactured by Sanko Junyaku Co., Ltd.), resulting in a cell concentration of 5 x $10^5$ cells/ml. Individual HTS FluoroBlok Inserts (inserts for 24-well plates; 3μm pore size; BD Falcon) were attached to a 24-well cell culture insert plate (BD Falcon). Thereafter, 100 μl of a suspension containing the fluorescently labeled cells was added to the inserts, and 600 μl of a growth medium for vascular endothelial cells (Bullet kit EGM-2, manufactured by Sanko Junyaku Co., Ltd.) which contained 10 ng/ml human VEGF (manufactured by R & D Systems Inc.), was added to the 24-well plate.

The cells which migrated through micropores of a filter, were observed and photographed by a fluorescence microscope from the bottom of the plate over time, up to 4 hours after the addition. The number of migrated cells was counted from the obtained images by using image analysis software (manufactured by Scion Image, Scion). As shown in Figure 7, the number of migrated cells in vascular endothelial cells transfected with KLF5 gene-specific siRNA No. 4, was smaller than that in vascular endothelial cells transfected with SEAP-siRNA used as a control. Thus, it was confirmed that siRNA capable of suppressing the expression of KLF5 gene is able to inhibit the migration of vascular endothelial cells.

Example 5 *In vivo* angiogenesis-inhibiting effect of siRNA capable of suppressing the expression of KLF5 gene

The *in vivo* angiogenesis-inhibiting effect of siRNA No.4 capable of suppressing the expression of KLF5 gene was examined by an assay using the Matrigel as shown below (Proc. Natl. Acad. Sci. USA, 94, 13612-13617, 1997; J. Biol. Chem., 277, 6667-6675, 2002).

A Matrigel mixture was prepared by adding 0.6 μg of mouse VEGF (manufactured by R & D Systems Inc.; Catalog No. 493-MV), 0.6 μg of bovine basic fibroblast growth factor (bFGF; manufactured by R & D Systems Inc.; Catalog No. 133-FB), and 10 μg of siRNA No. 4, to 0.5 ml (5 mg) of Matrigel Matrix (manufactured by BD Bioscience), and then mixing them via pipetting on ice. As a control, a Matrigel mixture containing SEAP-siRNA instead of siRNA No. 4 was also prepared. The thus prepared Matrigel mixture was subcutaneously injected into the back of a 6-week-old male C57BL/6 mouse. Fourteen days after the injection, the gelatinized Matrigel was removed therefrom. The obtained Matrigel was washed with PBS once and then immobilized with a 10% formaldehyde-PBS solution. The immobilized Matrigel was sliced into pieces with a thickness of 5 mm, and then embedded in a paraffin. Thereafter, the Matrigel sections were made by common histological means and then stained with hematoxylin and eosin. The stained Matrigel sections were observed under a microscope.

As a result, in the Matrigel to which SEAP-siRNA as a control had been added, a large number of vascular endothelial cells reacted with VEGF and bFGF added to the Matrigel, migrated, and infiltrated in Matrigel. In contrast, in the Matrigel to which siRNA No. 4 had been added, infiltration of vascular endothelial cells into the Matrigel was suppressed. Thus, it was confirmed that siRNA capable of suppressing the expression of KLF5 gene is able to inhibit angiogenesis.

Example 6 *In vivo* antitumor effect of siRNA that suppresses expression of KLF5 gene

The *in vivo* antitumor effect of siRNA No. 4 capable of suppressing the expression of KLF5 gene was examined using suppression of the growth of tumor as an indicator as follows.

The mouse Lewis lung carcinoma cell line LL/2 (obtained from Dainippon Pharmaceutical Co., Ltd.; Catalog No. 09-1642) was subcutaneously injected into the back of a 5-week-old male C57BL/6 mouse at a concentration of 1 x $10^6$ cells. Two days after the injection, immobilization of the Lewis lung carcinoma was confirmed, and siRNA No. 4 was then subcutaneously injected into the periphery of the cancer. As a control, SEAP-siRNA was also subcutaneously injected into the periphery of the cancer. With regard to the dosage of siRNA No. 4 and that of SEAP-siRNA, 1 μg of siRNA or SEAP-siRNA was dissolved in 50 μl of water for injection (Otsuka distilled water, manufactured by Otsuka Pharmaceutical Co., Ltd.), and the obtained solution was used per mouse. The above siRNA was administered to the mouse for 8 consecutive days. The number of administrations was once a day. The tumor volume after administration was calculated using the following formula (1), and an increase in the tumor volume was compared with the case of the control.

$$\text{Formula (1): tumor volume (mm}^3\text{)} = \{\text{tumor length (mm) x tumor width (mm)}^2\}/2$$

As a result, as shown in Figure 8, the tumor volume of a mouse to which SEAP-siRNA as a control had been administered increased after initiation of the administration thereof. In contrast, an increase in the tumor volume of a mouse to which siRNA No. 4 had been administered was suppressed from one day after the administration thereof. Thus, it was confirmed that siRNA capable of suppressing the expression of KLF5 gene has an *in vivo* antitumor effect, and that the growth of tumor can be suppressed by the administration thereof.

"Sequence Listing Free Text"

SEQ ID NO: 1 – Inventors: Ryozo Nagai; Ichiro Manabe; Atsushi Ishihara
Inventor: Tsuneaki Tottori

| | |
|---|---|
| SEQ ID NO: 17 | siRNA No. 1 sense strand |
| SEQ ID NO: 18 | siRNA No. 1 antisense strand |
| SEQ ID NO: 19 | siRNA No. 2 sense strand |
| SEQ ID NO: 20 | siRNA No. 2 antisense strand |
| SEQ ID NO: 21 | siRNA No. 3 sense strand |
| SEQ ID NO: 22 | siRNA No. 3 antisense strand |
| SEQ ID NO: 23 | siRNA No. 4 sense strand |
| SEQ ID NO: 24 | siRNA No. 4 antisense strand |
| SEQ ID NO: 25 | siRNA No. 5 sense strand |
| SEQ ID NO: 26 | siRNA No. 5 antisense strand |
| SEQ ID NO: 27 | siRNA No. 6 sense strand |
| SEQ ID NO: 28 | siRNA No. 6 antisense strand |
| SEQ ID NO: 29 | siRNA No. 7 sense strand |
| SEQ ID NO: 30 | siRNA No. 7 antisense strand |
| SEQ ID NO: 31 | siRNA No. 8 sense strand |
| SEQ ID NO: 32 | siRNA No. 8 antisense strand |
| SEQ ID NO: 33 | siRNA No. 9 sense strand |
| SEQ ID NO: 34 | siRNA No. 9 antisense strand |
| SEQ ID NO: 35 | siRNA No. 10 sense strand |
| SEQ ID NO: 36 | siRNA No. 10 antisense strand |
| SEQ ID NO: 37 | siRNA No. 11 sense strand |
| SEQ ID NO: 38 | siRNA No. 12 antisense strand |
| SEQ ID NO: 39 | siRNA-SEAP sense strand |
| SEQ ID NO: 40 | siRNA-SEAP antisense strand |
| SEQ ID NO: 41 | KLF5 gene-specific forward primer |
| SEQ ID NO: 42 | KLF5 gene-specific reverse primer |

| | |
|---|---|
| SEQ ID NO: 43 | PDGF-A gene-specific forward primer |
| SEQ ID NO: 44 | PDGF-A gene-specific reverse primer |
| SEQ ID NO: 45 | SMemb gene-specific forward primer |
| SEQ ID NO: 46 | SMemb gene-specific reverse primer |
| SEQ ID NO: 47 | SRF gene-specific forward primer |
| SEQ ID NO: 48 | SRF gene-specific reverse primer |

## Claims

1. An RNA capable of suppressing the expression of KLF5 gene, which comprises a sequence consisting of 15 to 30 contiguous nucleotides of KLF5 mRNA and a sequence complementary to the sequence.

2. The RNA according to claim 1, wherein the KLF5 mRNA is human KLF5 mRNA or mouse KLF5 mRNA.

3. The RNA according to claim 1 or 2, wherein the RNA is a double-stranded RNA consisting of a strand of a sequence consisting of 15 to 30 contiguous nucleotides of KLF5 mRNA and a strand of a sequence complementary to the sequence, in which 1 to 6 nucleotides are added to the 3'-terminus of each of the strands.

4. The RNA according to claim 1 or 2, wherein the RNA is an RNA forming a hairpin structure, which is obtained by ligating an RNA having a sequence consisting of 15 to 30 contiguous nucleotides of the KLF5 mRNA to an RNA having a sequence complementary to the sequence via a spacer oligonucleotide, and then adding 1 to 6 nucleotides to the 3'-terminus thereof.

5. An RNA capable of suppressing the expression of KLF5 gene, which is selected from the group consisting of the following (a) to (c):

   (a) a double-stranded RNA having a strand of a sequence shown in any one of SEQ ID NOS: 2 to 16 and a strand of a sequence complementary to the sequence, in which 2 to 4 uridylic acids or deoxythymidylic acids are added to the 3'-terminus of each of the strands;
   (b) an RNA forming a hairpin structure, which is obtained by ligating an RNA having a sequence shown in any one of SEQ ID NOS: 2 to 16 to an RNA having a sequence complementary to the sequence via spacer oligonucleotide that has 2 uridylic acids or deoxythymidylic acids at the 5'-terminus thereof, and then adding 2 to 4 uridylic acids or deoxythymidylic acids to the 3'-terminus thereof; and
   (c) a double-stranded RNA consisting of a strand of a sequence shown in any one of SEQ ID NOS: 2 to 11 and a strand of a sequence complementary to the sequence, in which 2 uridylic acids are added to the 3'-terminus of each of the strands.

6. A vector, which allows the RNA according to any one of claims 1 to 5 to be expressed.

7. A method of suppressing the expression of KLF5 gene in cells by transfecting the RNA according to any one of claims 1 to 5 or the vector according to claim 6 into the cells.

8. A method of suppressing the expression of a gene whose transcription is activated by KLF5 in cells by transfecting the RNA according to any one of claims 1 to 5 or the vector according to claim 6 into the cells.

9. The method according to claim 8, wherein the gene whose transcription is activated by KLF5 is platelet-derived growth factor A chain gene or a smooth muscle myosin heavy chain SMemb gene.

10. A pharmaceutical composition, which comprises, as an active ingredient, the RNA according to any one of claims 1 to 5 or the vector according to claim 6.

11. A pharmaceutical composition for inhibiting angiogenesis, which comprises, as an active ingredient, the RNA according to any one of claims 1 to 5 or the vector according to claim 6.

12. A therapeutic or preventive agent for cardiovascular disease or cancer, which comprises, as an active ingredient, the RNA according to any one of claims 1 to 5 or the vector according to claim 6.

13. The therapeutic or preventive agent according to claim 12, wherein the cardiovascular disease is arteriosclerosis, restenosis occurring after coronary intervention, or cardiac hypertrophy.

FIG.1

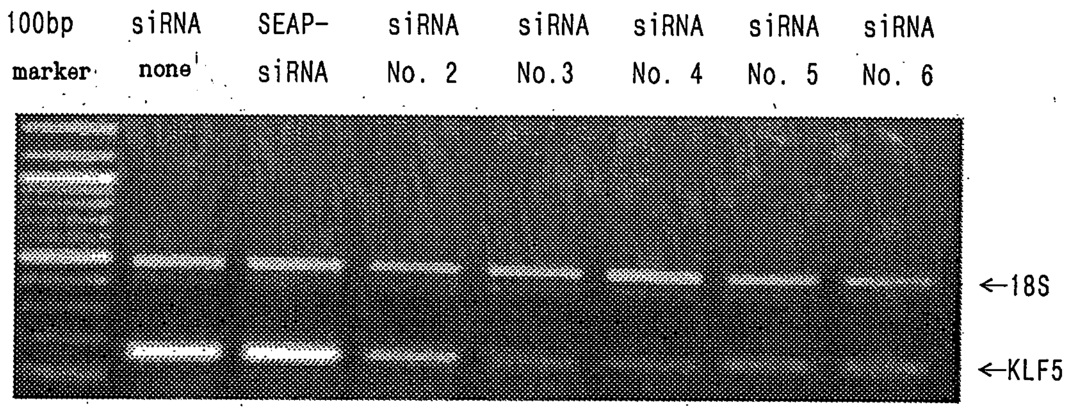
100bp marker
siRNA none
SEAP-siRNA
siRNA No. 2
siRNA No.3
siRNA No. 4
siRNA No. 5
siRNA No. 6
←18S
←KLF5

FIG.2

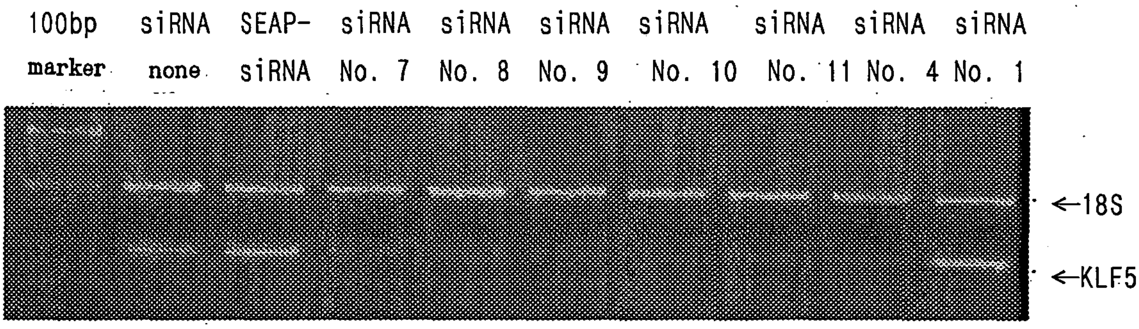
100bp marker
siRNA none
SEAP-siRNA
siRNA No. 7
siRNA No. 8
siRNA No. 9
siRNA No. 10
siRNA No. 11
siRNA No. 4
siRNA No. 1
←18S
←KLF5

FIG.3

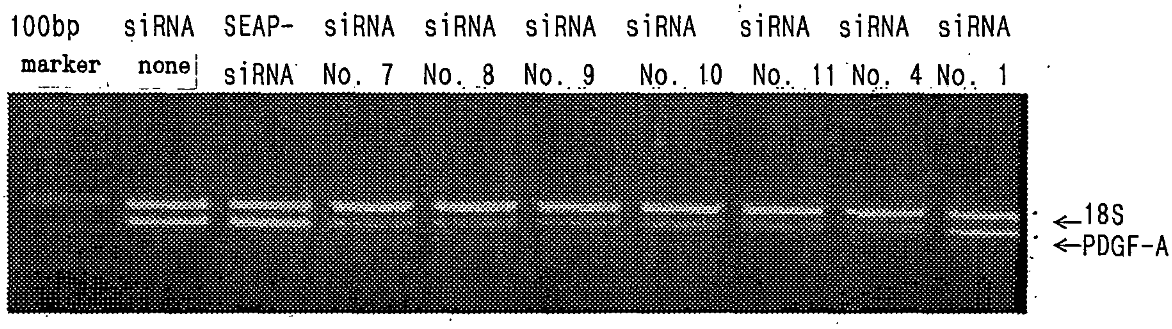
100bp marker
siRNA none
SEAP-siRNA
siRNA No. 7
siRNA No. 8
siRNA No. 9
siRNA No. 10
siRNA No. 11
siRNA No. 4
siRNA No. 1
←18S
←PDGF-A

FIG.4

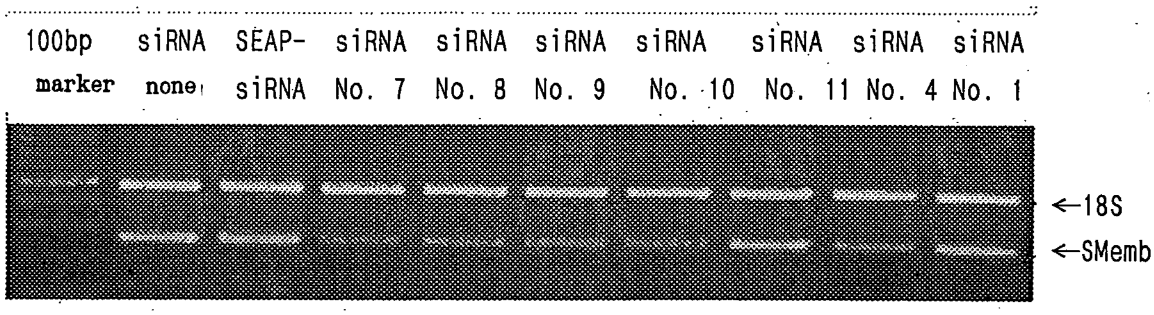
100bp marker
siRNA none
SEAP-siRNA
siRNA No. 7
siRNA No. 8
siRNA No. 9
siRNA No. 10
siRNA No. 11
siRNA No. 4
siRNA No. 1
←18S
←SMemb

FIG.5

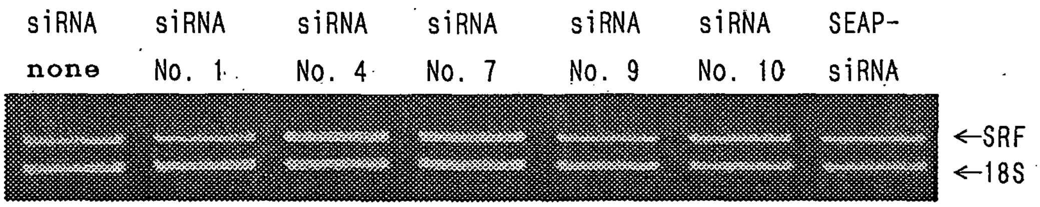
siRNA none
siRNA No. 1
siRNA No. 4
siRNA No. 7
siRNA No. 9
siRNA No. 10
SEAP-siRNA
←SRF
←18S

FIG.6

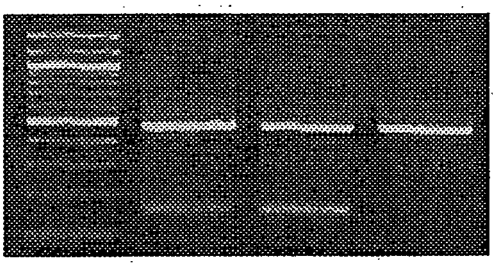

FIG.7

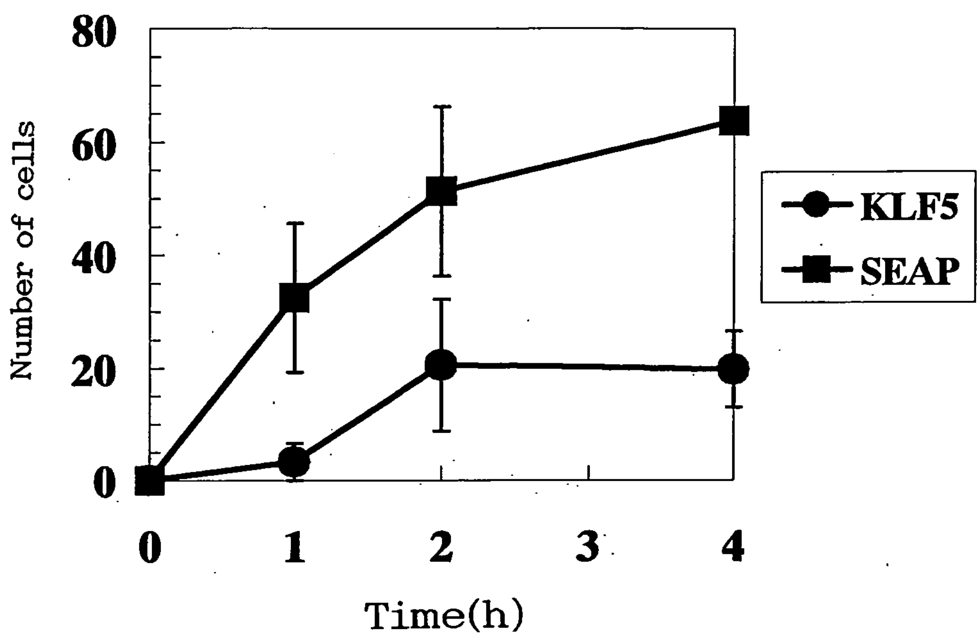

FIG.8

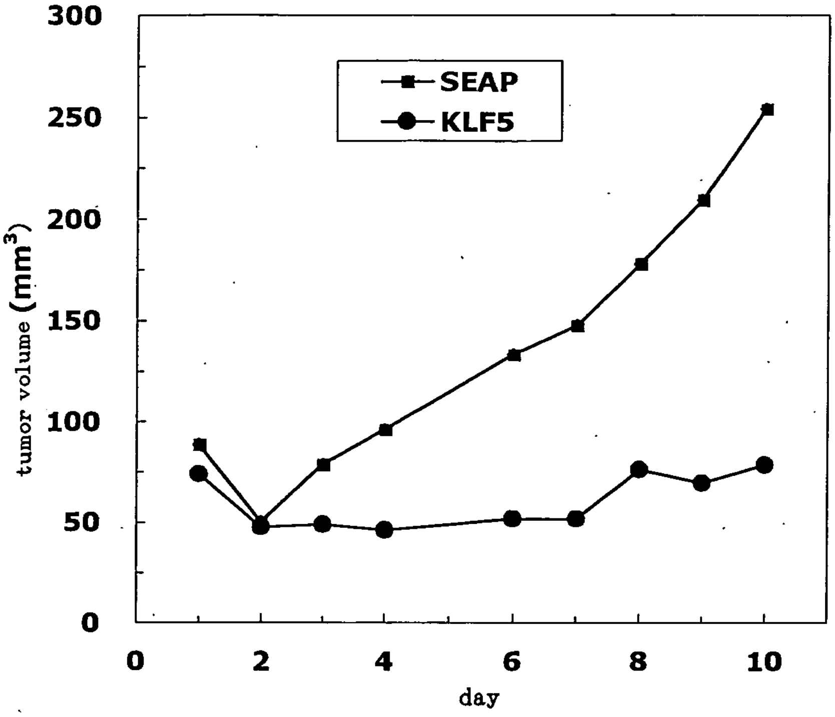
SEAP
KLF5
300
250
200
150
100
50
0
tumor volume (mm$^3$)
0
2
4
6
8
10
day

**INTERNATIONAL SEARCH REPORT**

International application No.
PCT/JP2004/011223

A. CLASSIFICATION OF SUBJECT MATTER
Int.Cl[7] C12N15/09, C12Q1/02, A61K48/00, A61P9/10, A61P35/00

According to International Patent Classification (IPC) or to both national classification and IPC

B. FIELDS SEARCHED

Minimum documentation searched (classification system followed by classification symbols)
Int.Cl[7] C12N15/09, C12Q1/02, A61K48/00, A61P9/10, A61P35/00

Documentation searched other than minimum documentation to the extent that such documents are included in the fields searched

Electronic data base consulted during the international search (name of data base and, where practicable, search terms used)
BIOSIS/WPI(DIALOG), MEDLINE(STN), JSTPlus/JST7580(JOIS)

C. DOCUMENTS CONSIDERED TO BE RELEVANT

| Category* | Citation of document, with indication, where appropriate, of the relevant passages | Relevant to claim No. |
|---|---|---|
| Y | Conkright M.D. et al., A gene encoding an intestinal-enriched member of the Kruppel-like factor family expressed in intestinal epithelial cells, Nucleic Acids Res, 1999, Vol.27, No.5, pages 1263 to 1270 | 1-13 |
| Y | Paul C.P. et al., Effective expression of small interfering RNA in human cells, Nat Biotechnol, 2002, Vol.20, No.5, pages 505 to 508 | 1-13 |
| Y | Hiroyuki OSHIUMI et al., "RNA interference (RNAi) o Mochiita Honyurui Dobutsu deno Idenshi Knockout", Folia Pharmacol.Jpn., 2002, Vol.120, pages 91 to 95 | 1-13 |

[X] Further documents are listed in the continuation of Box C.  [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 August, 2004 (20.08.04) | 07 September, 2004 (07.09.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/011223

C (Continuation). DOCUMENTS CONSIDERED TO BE RELEVANT

| Category* | Citation of document, with indication, where appropriate, of the relevant passages | Relevant to claim No. |
|---|---|---|
| Y | SHINDO, T. et al., Kruppel-like zinc-finger transcription factor KLF5/BTEB2 is a target for angiotensin II signaling and an essential regulator of cardiovascular remodeling, Nat Med, 2002, Vol.8, No.8, pages 856 to 863 | 9-13 |

Form PCT/ISA/210 (continuation of second sheet) (January 2004)

**INTERNATIONAL SEARCH REPORT**

International application No.
PCT/JP2004/011223

**Box No. I Nucleotide and/or amino acid sequence(s) (Continuation of item1.b of the first sheet)**

1. With regard to any nucleotide and/or amino acid sequence disclosed in the international application and necessary to the claimed invention, the international search was carried out on the basis of:

   a. type of material
      - ☐ a sequence listing
      - ☒ table(s) related to the sequence listing

   b. format of material
      - ☐ in written format
      - ☒ in computer readable form

   c. time of filing/furnishing
      - ☐ contained in the international application as filed
      - ☒ filed together with the international application in computer readable form
      - ☐ furnished subsequently to this Authority for the purposes of search

2. ☐ In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (January 2004)

SEQUENCE LISTING

<110> Nagai, Ryozo; Manabe, Ichiro; Kyowa Hakko Kogyo Co., Ltd.

<120> RNAs which inhibit KLF5 gene expression

<130> 1596

<150> JP 2003-202863
<151> 2003-07-29

<150> JP 2004-075115
<151> 2004-03-16

<160> 50

<170> PatentIn version 3.1

<210> 1
<211> 19
<212> RNA
<213> Mus musculus

<220>
<223> Inventor: Nagai, Ryozo; Manabe, Ichiro; Ishihara, Atsushi;
Inventor: Tottori, Tsuneaki

<400> 1
caugaacguc uuccucccu 19

<210> 2
<211> 19
<212> RNA
<213> Mus musculus

<400> 2
auuuaccugc cacucugcc 19

<210> 3
<211> 19
<212> RNA
<213> Mus musculus

<400> 3
ggaguaaccc ggaucugga 19

<210> 4
<211> 19
<212> RNA
<213> Mus musculus

<400> 4
aagcucaccu gaggacuca 19

<210> 5
<211> 19
<212> RNA
<213> Mus musculus

<400> 5
uccccagacc guccaugcc 19

<210> 6
<211> 19
<212> RNA
<213> Mus musculus

<400> 6
cgcugcgccc acccgccug 19

<210> 7
<211> 19
<212> RNA
<213> Mus musculus

<400> 7
auggagaagu aucugaccc 19

<210> 8
<211> 19
<212> RNA
<213> Mus musculus

<400> 8
aguauagacg agacagugc 19

<210> 9
<211> 19
<212> RNA
<213> Mus musculus

<400> 9
accagacggc aguaaugga 19

<210> 10
<211> 19
<212> RNA

<213> Mus musculus

<400> 10
gcucagagcc uggaagucc 19

<210> 11
<211> 19
<212> RNA
<213> Mus musculus

<400> 11
gccguuccag ugcauggug 19

<210> 12
<211> 19
<212> RNA
<213> Homo sapiens

<400> 12
auuuacccac cacccugcc 19

<210> 13
<211> 19
<212> RNA
<213> Homo sapiens

<400> 13
ggaguaaccc cgauuugga 19

<210> 14
<211> 19
<212> RNA
<213> Homo sapiens

<400> 14
auggagaagu aucugacac 19

<210> 15
<211> 19
<212> RNA
<213> Homo sapiens

<400> 15
aucagacagc agcaaugga 19

<210> 16
<211> 19

<212> RNA
<213> Homo sapiens

<400> 16
gcccuuccag ugcggggug 19

<210> 17
<211> 21
<212> RNA
<213> Artificial

<220>
<223> siRNA No. 1 sense strand

<220>
<221> misc_feature
<222> (20)..(21)
<223> DNA

<400> 17
caugaacguc uuccucccut t 21

<210> 18
<211> 21
<212> RNA
<213> Artificial

<220>
<223> siRNA No. 1 antisense strand

<220>
<221> misc_feature
<222> (20)..(21)
<223> DNA

<400> 18
agggaggaag acguucaugt t 21

<210> 19
<211> 21
<212> RNA
<213> Artificial

<220>
<223> siRNA No. 2 sense strand

<400> 19
auuuaccugc cacucugccu u 21

<210> 20
<211> 21
<212> RNA
<213> Artificial

<220>
<223> siRNA No. 2 antisense strand

<400> 20
ggcagagugg cagguaaauu u 21

<210> 21
<211> 21
<212> RNA
<213> Artificial

<220>
<223> siRNA No. 3 sense strand

<400> 21
ggaguaaccc ggaucuggau u 21

<210> 22
<211> 21
<212> RNA
<213> Artificial

<220>
<223> siRNA #3 antisense strand

<400> 22
uccagauccg gguuacuccu u 21

<210> 23
<211> 21
<212> RNA
<213> Artificial

<220>
<223> siRNA No. 4 sense strand

<400> 23
aagcucaccu gaggacucau u 21

<210> 24
<211> 21
<212> RNA
<213> Artificial

<220>
<223> siRNA No. 4 antisense strand

<400> 24
ugaguccuca ggugagcuuu u 21

<210> 25
<211> 21
<212> RNA
<213> Artificial

<220>
<223> siRNA No. 5 sense strand

<400> 25
uccccagacc guccaugccu u 21

<210> 26
<211> 21
<212> RNA
<213> Artificial

<220>
<223> siRNA No. 5 antisense strand

<400> 26
ggcauggacg gucuggggu u 21

<210> 27
<211> 21
<212> RNA
<213> Artificial

<220>
<223> siRNA No. 6 sense strand

<400> 27
cgcugcgccc acccgccugu u 21

<210> 28
<211> 21
<212> RNA
<213> Artificial

<220>
<223> siRNA No. 6 antisense strand

<400> 28
caggcgggug ggcgcagcgu u 21

<210> 29
<211> 21
<212> RNA
<213> Artificial

<220>
<223> siRNA No. 7 sense strand

<400> 29
auggagaagu aucugacccu u 21

<210> 30
<211> 21
<212> RNA
<213> Artificial

<220>
<223> siRNA No. 7 antisense strand

<400> 30
gggucagaua cuucuccauu u 21

<210> 31
<211> 21
<212> RNA
<213> Artificial

<220>
<223> siRNA No. 8 sense strand

<400> 31
aguauagacg agacagugcu u 21

<210> 32
<211> 21
<212> RNA
<213> Artificial

<220>
<223> siRNA No. 8 antisense strand

<400> 32
gcacugucuc gucuauacuu u 21

<210> 33
<211> 21
<212> RNA

<213> Artificial

<220>
<223> siRNA No. 9 sense strand

<400> 33
accagacggc aguaauggau u 21

<210> 34
<211> 21
<212> RNA
<213> Artificial

<220>
<223> siRNA No. 9 antisense strand

<400> 34
uccauuacug ccgucuggcu u 21

<210> 35
<211> 21
<212> RNA
<213> Artificial

<220>
<223> siRNA No. 10 sense strand

<400> 35
gcucagagcc uggaaguccu u 21

<210> 36
<211> 21
<212> RNA
<213> Artificial

<220>
<223> siRNA No. 10 antisense strand

<400> 36
ggacuuccag gcucugagcu u 21

<210> 37
<211> 21
<212> RNA
<213> Artificial

<220>
<223> siRNA No. 11 sense strand

<400> 37
gccguuccag ugcauggugu u 21

<210> 38
<211> 21
<212> RNA
<213> Artificial

<220>
<223> siRNA No. 11 antisense strand

<400> 38
caccaugcac uggaacggcu u 21

<210> 39
<211> 21
<212> RNA
<213> Artificial

<220>
<223> SEAP-siRNA sense strand

<400> 39
agggcaacuu ccagaccauu u 21

<210> 40
<211> 21
<212> RNA
<213> Artificial

<220>
<223> SEAP-siRNA antisense strand

<400> 40
auggucugga aguugcccuu u 21

<210> 41
<211> 20
<212> DNA
<213> Artificial

<220>
<223> KLF5 gene specific forward primer

<400> 41
ggttgcacaa aagtttatac 20

<210> 42

<211> 22
<212> DNA
<213> Artificial

<220>
<223> KLF5 gene specific riverse primer

<400> 42
ggcttggcgc ccgtgtgctt cc 22

<210> 43
<211> 22
<212> DNA
<213> Artificial

<220>
<223> PDGF-A gene specific forward primer

<400> 43
ctccagcgac tcttggagat ag 22

<210> 44
<211> 22
<212> DNA
<213> Artificial

<220>
<223> PDGF-A gene specific riverse primer

<400> 44
ttcaggttgg aggtcgcaca tg 22

<210> 45
<211> 25
<212> DNA
<213> Artificial

<220>
<223> SMemb gene specific forward primer

<400> 45
aatgcccgcc agcagctgga gcgac 25

<210> 46
<211> 25
<212> DNA
<213> Artificial

<220>

<223> SMemb gene specific riverse primer

<400> 46
gctccttata ctgatccgca tgccg 25

<210> 47
<211> 25
<212> DNA
<213> Artificial

<220>
<223> SRF gene specific forward primer

<400> 47
tggcaccagt gtctgctact gtcag 25

<210> 48
<211> 25
<212> DNA
<213> Artificial

<220>
<223> SRF gene specific riverse primer

<400> 48
gctgccctat cacagccatc tggtg 25

<210> 49
<211> 1591
<212> DNA
<213> Mus musculus

<220>
<221> CDS
<222> (167)..(1507)
<223>

<400> 49
ccgagcccag gagccccgat ctccgtgccc gccttcgtga gcgtctggct gccggcccag 60

gggtcccccg ccgcggcccc ccgccgagtc cgccgtcccg tgccagcccg agcgaggtgg 120

gatcgcgatc gctccgtgtc ccgctcccgt aatccccaga ccgtcc atg ccc acg 175
Met Pro Thr
1

cgg gtg ctg acc atg agc gcc cgc ctg gga cca ctg ccc cag ccg ccg 223
Arg Val Leu Thr Met Ser Ala Arg Leu Gly Pro Leu Pro Gln Pro Pro
5 10 15

```
gcc gcg cag gcc gag ccc gtg ttc gcg cag ctc aag ccg gtg ctg ggc       271
Ala Ala Gln Ala Glu Pro Val Phe Ala Gln Leu Lys Pro Val Leu Gly
20                  25                  30                  35

gct gcg aac ccg gcc cgc gac gcg gcg ctc ttc tcc gga gac gat ctg       319
Ala Ala Asn Pro Ala Arg Asp Ala Ala Leu Phe Ser Gly Asp Asp Leu
                40                  45                  50

aaa cac gcg cac cac cac ccg cct gcg ccg ccg cca gcc gct ggc ccg       367
Lys His Ala His His His Pro Pro Ala Pro Pro Pro Ala Ala Gly Pro
            55                  60                  65

cga ctg ccc tcg gag gag ctg gtc cag aca aga tgt gaa atg gag aag       415
Arg Leu Pro Ser Glu Glu Leu Val Gln Thr Arg Cys Glu Met Glu Lys
        70                  75                  80

tat ctg acc cct cag ctc cct cca gtt ccg ata att tca gag cat aaa       463
Tyr Leu Thr Pro Gln Leu Pro Pro Val Pro Ile Ile Ser Glu His Lys
    85                  90                  95

aag tat aga cga gac agt gcc tca gtg gta gac cag ttc ttc act gac       511
Lys Tyr Arg Arg Asp Ser Ala Ser Val Val Asp Gln Phe Phe Thr Asp
100                 105                 110                 115

act gaa ggc ata cct tac agc atc aac atg aac gtc ttc ctc cct gac       559
Thr Glu Gly Ile Pro Tyr Ser Ile Asn Met Asn Val Phe Leu Pro Asp
                120                 125                 130

atc act cac ctg aga act ggc ctc tac aaa tcc cag aga cca tgc gta       607
Ile Thr His Leu Arg Thr Gly Leu Tyr Lys Ser Gln Arg Pro Cys Val
            135                 140                 145

aca cag atc aag aca gaa cct gtt acc att ttc agc cac cag agc gag       655
Thr Gln Ile Lys Thr Glu Pro Val Thr Ile Phe Ser His Gln Ser Glu
        150                 155                 160

tcg acg gcc cct cct cct cct ccg gcc ccc acc cag gct ctc ccc gag       703
Ser Thr Ala Pro Pro Pro Pro Pro Ala Pro Thr Gln Ala Leu Pro Glu
    165                 170                 175

ttc act agt atc ttc agc tcc cac cag acc aca gcg cca cca cag gag       751
Phe Thr Ser Ile Phe Ser Ser His Gln Thr Thr Ala Pro Pro Gln Glu
180                 185                 190                 195

gtg aac aat atc ttc atc aaa caa gaa ctt cct ata cca gat ctt cat       799
Val Asn Asn Ile Phe Ile Lys Gln Glu Leu Pro Ile Pro Asp Leu His
                200                 205                 210

ctc tct gtc cct tcc cag cag ggc cac ctg tac cag ctg ttg aat aca       847
Leu Ser Val Pro Ser Gln Gln Gly His Leu Tyr Gln Leu Leu Asn Thr
            215                 220                 225

ccg gat cta gac atg ccc agt tcg aca aac cag acg gca gta atg gac       895
```

```
Pro Asp Leu Asp Met Pro Ser Ser Thr Asn Gln Thr Ala Val Met Asp
        230                 235                 240

acc ctt aat gtt tct atg gca ggc ctt aac cca cac ccc tct gct gtt      943
Thr Leu Asn Val Ser Met Ala Gly Leu Asn Pro His Pro Ser Ala Val
    245                 250                 255

cca cag acg tca atg aaa cag ttc cag ggc atg ccc cct tgc acg tac      991
Pro Gln Thr Ser Met Lys Gln Phe Gln Gly Met Pro Pro Cys Thr Tyr
260                 265                 270                 275

acc atg cca agt cag ttt ctt cca cag cag gcc act tat ttc ccc ccg     1039
Thr Met Pro Ser Gln Phe Leu Pro Gln Gln Ala Thr Tyr Phe Pro Pro
                280                 285                 290

tca cca cca agc tca gag cct gga agt ccc gat aga caa gct gag atg     1087
Ser Pro Pro Ser Ser Glu Pro Gly Ser Pro Asp Arg Gln Ala Glu Met
            295                 300                 305

ctg cag aat ctc acc cca cct ccg tcc tat gcc gct aca att gct tcc     1135
Leu Gln Asn Leu Thr Pro Pro Pro Ser Tyr Ala Ala Thr Ile Ala Ser
        310                 315                 320

aaa ctg gcg att cac aac cca aat tta cct gcc act ctg cca gtt aat     1183
Lys Leu Ala Ile His Asn Pro Asn Leu Pro Ala Thr Leu Pro Val Asn
    325                 330                 335

tcg cca act ctc cca cct gtc aga tac aac aga agg agt aac ccg gat     1231
Ser Pro Thr Leu Pro Pro Val Arg Tyr Asn Arg Arg Ser Asn Pro Asp
340                 345                 350                 355

ctg gag aag cga cgt atc cac ttc tgc gat tat aat ggt tgc aca aaa     1279
Leu Glu Lys Arg Arg Ile His Phe Cys Asp Tyr Asn Gly Cys Thr Lys
                360                 365                 370

gtt tat aca aag tcg tct cac tta aaa gct cac ctg agg act cat acg     1327
Val Tyr Thr Lys Ser Ser His Leu Lys Ala His Leu Arg Thr His Thr
            375                 380                 385

ggc gag aag ccc tac aag tgc acc tgg gag ggc tgc gac tgg agg ttt     1375
Gly Glu Lys Pro Tyr Lys Cys Thr Trp Glu Gly Cys Asp Trp Arg Phe
        390                 395                 400

gcc cgg tcg gat gag ctg acc cgc cac tac agg aag cac acg ggc gcc     1423
Ala Arg Ser Asp Glu Leu Thr Arg His Tyr Arg Lys His Thr Gly Ala
    405                 410                 415

aag ccg ttc cag tgc atg gtg tgc caa cgc agc ttc tcc cgc tcc gac     1471
Lys Pro Phe Gln Cys Met Val Cys Gln Arg Ser Phe Ser Arg Ser Asp
420                 425                 430                 435

cac ctc gcg ctg cac atg aag cgc cac cag aac tga gcgagcgaac          1517
His Leu Ala Leu His Met Lys Arg His Gln Asn
```

440 445

gctgcgccca cccgcctgac gccttgcagt ccgctttgcc atcctttaaa ccgcagacct 1577

aacttcataa aaag 1591

<210> 50
<211> 3359
<212> DNA
<213> Homo sapiens

<220>
<221> CDS
<222> (312)..(1685)

<400> 50
ggtacgtgcg ctcgcggttc tctcgcggag gtcggcggtg gcgggagcgg gctccggaga 60

gcctgagagc acggtggggc ggggcgggag aaagtggccg cccggaggac gttggcgttt 120

acgtgtggaa gagcggaaga gttttgcttt tcgtgcgcgc cttcgaaaac tgcctgccgc 180

tgtctgagga gtccacccga aacctcccct cctccgccgg cagccccgcg ctgagctcgc 240

cgacccaagc cagcgtgggc gaggtgggaa gtgcgcccga cccgcgcctg gagctgcgcc 300

cccgagtgcc c atg gct aca agg gtg ctg agc atg agc gcc cgc ctg gga 350
Met Ala Thr Arg Val Leu Ser Met Ser Ala Arg Leu Gly
1 5 10

ccc gtg ccc cag ccg ccg gcg ccg cag gac gag ccg gtg ttc gcg cag 398
Pro Val Pro Gln Pro Pro Ala Pro Gln Asp Glu Pro Val Phe Ala Gln
15 20 25

ctc aag ccg gtg ctg ggc gcc gcg aat ccg gcc cgc gac gcg gcg ctc 446
Leu Lys Pro Val Leu Gly Ala Ala Asn Pro Ala Arg Asp Ala Ala Leu
30 35 40 45

ttc ccc ggc gag gag ctg aag cac gcg cac cac cgc ccg cag gcg cag 494
Phe Pro Gly Glu Glu Leu Lys His Ala His His Arg Pro Gln Ala Gln
50 55 60

ccc gcg ccc gcg cag gcc ccg cag ccg gcc cag ccg ccc gcc acc ggc 542
Pro Ala Pro Ala Gln Ala Pro Gln Pro Ala Gln Pro Pro Ala Thr Gly
65 70 75

ccg cgg ctg cct cca gag gac ctg gtc cag aca aga tgt gaa atg gag 590
Pro Arg Leu Pro Pro Glu Asp Leu Val Gln Thr Arg Cys Glu Met Glu
80 85 90

aag tat ctg aca cct cag ctt cct cca gtt cct ata att cca gag cat 638
Lys Tyr Leu Thr Pro Gln Leu Pro Pro Val Pro Ile Ile Pro Glu His

```
    95                  100                 105

aaa aag tat aga cga gac agt gcc tca gtc gta gac cag ttc ttc act        686
Lys Lys Tyr Arg Arg Asp Ser Ala Ser Val Val Asp Gln Phe Phe Thr
110                 115                 120                 125

gac act gaa ggg tta cct tac agt atc aac atg aac gtc ttc ctc cct        734
Asp Thr Glu Gly Leu Pro Tyr Ser Ile Asn Met Asn Val Phe Leu Pro
                130                 135                 140

gac atc act cac ctg aga act ggc ctc tac aaa tcc cag aga ccg tgc        782
Asp Ile Thr His Leu Arg Thr Gly Leu Tyr Lys Ser Gln Arg Pro Cys
            145                 150                 155

gta aca cac atc aag aca gaa cct gtt gcc att ttc agc cac cag agt        830
Val Thr His Ile Lys Thr Glu Pro Val Ala Ile Phe Ser His Gln Ser
        160                 165                 170

gaa acg act gcc cct cct ccg gcc ccg acc cag gcc ctc cct gag ttc        878
Glu Thr Thr Ala Pro Pro Pro Ala Pro Thr Gln Ala Leu Pro Glu Phe
    175                 180                 185

acc agt ata ttc agc tca cac cag acc gca gct cca gag gtg aac aat        926
Thr Ser Ile Phe Ser Ser His Gln Thr Ala Ala Pro Glu Val Asn Asn
190                 195                 200                 205

att ttc atc aaa caa gaa ctt cct aca cca gat ctt cat ctt tct gtc        974
Ile Phe Ile Lys Gln Glu Leu Pro Thr Pro Asp Leu His Leu Ser Val
                210                 215                 220

cct acc cag cag ggc cac ctg tac cag cta ctg aat aca ccg gat cta       1022
Pro Thr Gln Gln Gly His Leu Tyr Gln Leu Leu Asn Thr Pro Asp Leu
            225                 230                 235

gat atg ccc agt tct aca aat cag aca gca gca atg gac act ctt aat       1070
Asp Met Pro Ser Ser Thr Asn Gln Thr Ala Ala Met Asp Thr Leu Asn
        240                 245                 250

gtt tct atg tca gct gcc atg gca ggc ctt aac aca cac acc tct gct       1118
Val Ser Met Ser Ala Ala Met Ala Gly Leu Asn Thr His Thr Ser Ala
    255                 260                 265

gtt ccg cag act gca gtg aaa caa ttc cag ggc atg ccc cct tgc aca       1166
Val Pro Gln Thr Ala Val Lys Gln Phe Gln Gly Met Pro Pro Cys Thr
270                 275                 280                 285

tac aca atg cca agt cag ttt ctt cca caa cag gcc act tac ttt ccc       1214
Tyr Thr Met Pro Ser Gln Phe Leu Pro Gln Gln Ala Thr Tyr Phe Pro
                290                 295                 300

ccg tca cca cca agc tca gag cct gga agt cca gat aga caa gca gag       1262
Pro Ser Pro Pro Ser Ser Glu Pro Gly Ser Pro Asp Arg Gln Ala Glu
            305                 310                 315
```

```
atg ctc cag aat tta acc cca cct cca tcc tat gct gct aca att gct      1310
Met Leu Gln Asn Leu Thr Pro Pro Pro Ser Tyr Ala Ala Thr Ile Ala
        320                 325                 330

tct aaa ctg gca att cac aat cca aat tta ccc acc acc ctg cca gtt      1358
Ser Lys Leu Ala Ile His Asn Pro Asn Leu Pro Thr Thr Leu Pro Val
    335                 340                 345

aac tca caa aac atc caa cct gtc aga tac aat aga agg agt aac ccc      1406
Asn Ser Gln Asn Ile Gln Pro Val Arg Tyr Asn Arg Arg Ser Asn Pro
350                 355                 360                 365

gat ttg gag aaa cga cgc atc cac tac tgc gat tac cct ggt tgc aca      1454
Asp Leu Glu Lys Arg Arg Ile His Tyr Cys Asp Tyr Pro Gly Cys Thr
                370                 375                 380

aaa gtt tat acc aag tct tct cat tta aaa gct cac ctg agg act cac      1502
Lys Val Tyr Thr Lys Ser Ser His Leu Lys Ala His Leu Arg Thr His
            385                 390                 395

act ggt gaa aag cca tac aag tgt acc tgg gaa ggc tgc gac tgg agg      1550
Thr Gly Glu Lys Pro Tyr Lys Cys Thr Trp Glu Gly Cys Asp Trp Arg
        400                 405                 410

ttc gcg cga tcg gat gag ctg acc cgc cac tac cgg aag cac aca ggc      1598
Phe Ala Arg Ser Asp Glu Leu Thr Arg His Tyr Arg Lys His Thr Gly
    415                 420                 425

gcc aag ccc ttc cag tgc ggg gtg tgc aac cgc agc ttc tcg cgc tct      1646
Ala Lys Pro Phe Gln Cys Gly Val Cys Asn Arg Ser Phe Ser Arg Ser
430                 435                 440                 445

gac cac ctg gcc ctg cat atg aag agg cac cag aac tga gcactgcccg       1695
Asp His Leu Ala Leu His Met Lys Arg His Gln Asn
                450                 455

tgtgacccgt tccaggtccc ctgggctccc tcaaatgaca gacctaacta ttcctgtgta    1755

aaaacaacaa aaacaaaaaa aaaacaagaa aaccacaact aaaactggaa atgtatattt    1815

tgtatatttg agaaaacagg gaatacattg tattaatacc aaagtgtttg gtcattttaa    1875

gaatctggaa tgcttgctgt aatgtatatg gctttactca agcagatctc atctcatctc    1935

atgacaggca gccagtctca acatgggtaa ggggtggggg tgaaggggag tgtgtgcagc    1995

gtttttacct aggcaccatc atttaatgtg acagtgttca gtaaacaaat cagttggcag    2055

gcaccagaag aagaatggat tgtatgtcaa gattttactt ggcattgagt agttttttc     2115

aatagtaggt aattccttag agatacagta tacctggcaa ttcacaaata gccattgaac    2175
```

aaatgtgtgg gtttttaaaa attatataca tatatgagtt gcctatattt gctattcaaa 2235
atttgtaaa tatgcaaatc agctttatag gtttattaca agttttttag gattcttttg 2295
gggaagagtc ataattcttt tgaaaataac catgaataca cttacagtta ggatttgtgg 2355
taaggtacct ctcaacatta ccaaaatcat ttctttagag ggaaggaata atcattcaaa 2415
tgaactttaa aaaagcaaat ttcatgcact gattaaaata ggattatttt aaatacaaaa 2475
ggcattttat atgaattata aactgaagag cttaaagata gttacaaaat acaaaagttc 2535
aacctcttac aataagctaa acgcaatgtc atttttaaaa agaaggactt aggggtcgtt 2595
ttcacatatg acaatgttgc atttatgatg cagttttcaa gtaccaaaac gttgaattga 2655
tgatgcagtt ttcatatatc gagatgttcg ctcgtgcagt actgttggtt aaatgacaat 2715
ttatgtggat tttgcatgta atacacagtg agacacagta attttatcta aattacagtg 2775
cagtttagtt aatctattaa tactgactca gtgtctgcct ttaaatataa atgatatgtt 2835
gaaaacttaa ggaagcaaat gctacatata tgcaatataa aatagtaatg tgatgctgat 2895
gctgttaacc aaagggcaga ataaataagc aaaatgccaa aaggggtctt aattgaaatg 2955
aaaatttaat tttgttttta aaatattgtt tatctttatt tattttgtgg taatatagta 3015
agttttttta gaagacaatt ttcataactt gataaattat agttttgttt gttagaaaag 3075
ttgctcttaa aagatgtaaa tagatgacaa acgatgtaaa taattttgta agaggcttca 3135
aaatgtttat acgtggaaac acacctacat gaaaagcaga aatcggttgc tgttttgctt 3195
cttttccct cttattttg tattgtggtc atttcctatg caaataatgg agcaaacagc 3255
tgtatagttg tagaattttt tgagagaatg agatgtttat atattaacga caattttttt 3315
tttggaaaat aaaaagtgcc taaaagaaaa aaaaaaaaaa aaaa 3359